# EUROPEAN PATENT APPLICATION

(11) **EP 1 296 138 A1**
(43) Date of publication of application: **26.03.2003**
(21) Application number: 01945720.9
(22) Date of filing: 29.06.2001
(51) Int. Cl.: G01N 33/50, G01N 33/53, A61K 45/00, A61P 9/00, A61K 31/711, A61K 38/17, A61K 39/395, A61K 48/00, C12N 15/12

(54) **USE OF DISEASE-ASSOCIATED GENE**

(30) Priority: 30.06.2000 JP 2000203104
(71) Applicant: Takeda Chemical Industries, Ltd., Osaka-shi, Osaka 541-0045 (JP)
(72) Inventor: KOYAMA, Nobuyuki, Tsukuba-shi, Ibaraki 305-0821 (JP); TANIDA, Seiichi, Nagaokakyo-shi, Kyoto 617-0847 (JP); WATANABE, Toshifumi, Kawachinagano-shi, Osaka 586-0092 (JP)
(74) Representative: Lewin, John Harvey
(86) International application number: JP0105652
(87) International publication number: WO02003063

(57) **Abstract**

The present invention relates to a method of screening a drug by using a disease-associated gene product, an antibody to the disease-associated gene product, an antisense DNA that suppresses the expression of the disease-associated gene, etc.

A compound regulating the activity of a protein having an amino acid sequence which is the same or substantially the same amino acid sequence as the amino acid sequence represented by SEQ ID NO:1, or its salt, a neutralizing antibody regulating the activity of the above protein, an antisense DNA, etc. can regulate the expression of the protein having an amino acid sequence which is the same or substantially the same amino acid sequence as the amino acid sequence represented by SEQ ID NO: 1 and, therefore, are usable as preventive/therapeutic agents for diseases, e.g., heart diseases, etc.

SEQUENCE LISTING

<110> Takeda Chemical Industries, Ltd.

<120> Use of Disease-Related Gene

<130> 2748WOOP

<150> JP 2000-203104

<151> 2000-06-30

<160> 20

<210> 1

<211> 319

<212> PRT

<213> Rat

<400> 1

Met Met Val Phe Arg Val Glu Glu Leu Val Thr Gly Lys Lys Asn Ser
                5                   10                  15
Asn Gly Ser Ser Gly Glu Phe Leu Pro Gly Glu Phe Arg Asn Gly Glu
            20                  25                  30
Tyr Glu Ala Ala Val Ala Leu Glu Lys Gln Glu Asp Leu Lys Thr Leu
        35                  40                  45
Pro Ala Asn Ser Val Asn Leu Gly Glu Glu Gln Arg Lys Ser Glu Lys
    50                  55                  60
Val Arg Glu Ala Glu Leu Lys Lys Lys Lys Leu Glu Gln Arg Ser Lys
65                  70                  75                  80
Leu Glu Asn Leu Glu Asp Leu Glu Ile Ile Val Gln Leu Lys Lys Arg
                85                  90                  95
Lys Lys Tyr Lys Lys Thr Lys Val Pro Val Val Lys Glu Pro Glu Pro
            100                 105                 110
Glu Ile Ile Thr Glu Pro Val Asp Val Pro Arg Phe Leu Lys Ala Ala
        115                 120                 125

Leu Glu Asn Lys Leu Pro Val Val Glu Lys Phe Leu Ser Asp Lys Asn
    130                 135                 140
Ser Pro Asp Val Cys Asp Glu Tyr Lys Arg Thr Ala Leu His Arg Ala
145                 150                 155                 160
Cys Leu Glu Gly His Leu Ala Ile Val Glu Lys Leu Met Glu Ala Gly
                165                 170                 175
Ala Gln Ile Glu Phe Arg Asp Met Leu Glu Ser Thr Ala Ile His Trp
            180                 185                 190
Ala Cys Arg Gly Gly Asn Leu Asp Val Leu Lys Leu Leu Leu Asn Lys
        195                 200                 205
Gly Ala Lys Ile Ser Ala Arg Asp Lys Leu Leu Ser Thr Ala Leu His
    210                 215                 220
Val Ala Val Arg Thr Gly His Tyr Glu Cys Ala Glu His Leu Ile Ala
225                 230                 235                 240
Cys Glu Ala Asp Leu Asn Ala Lys Asp Arg Glu Gly Asp Thr Pro Leu
                245                 250                 255
His Asp Ala Val Arg Leu Asn Arg Tyr Lys Met Ile Arg Leu Leu Met
            260                 265                 270
Thr Phe Gly Ala Asp Leu Asn Val Lys Asn Cys Ala Gly Lys Thr Pro
        275                 280                 285
Met Asp Leu Val Leu His Trp Gln Asn Gly Thr Lys Ala Ile Phe Asp
    290                 295                 300
Ser Leu Lys Glu Asn Ala Tyr Lys Asn Ser Arg Ile Ala Thr Phe
305                 310                 315
<210> 2
<211> 957
<212> DNA
<213> Rat
<400> 2

atgatggttt ttcgagtaga ggagctggta acgggcaaaa agaacagcaa tgggtcctca 60
ggggagttcc ttcctggcga gttcagaaat ggagagtatg aagctgctgt tgccttggag 120
aaacaagagg acttgaagac acttccagcc aacagtgtga acctggggga agaacaacgg 180
aaaagtgaga aagttcgaga ggcagagctc aaaaagaaaa aactagaaca aagatcaaag 240
cttgaaaact tagaagacct tgaaataatt gttcaactga agaaaagaaa aaaatacaag 300
aaaactaaag ttccagttgt gaaggaaccg gagcctgaaa ttattactga acctgtggat 360
gtgccaaggt ttctgaaagc tgcactggag aacaaactgc cagttgtaga gaaattcctg 420
tcagacaaga acagccccga cgtctgcgat gagtataaac ggaccgctct ccatagagca 480
tgcttagaag gacacttggc gatcgtggag aagttaatgg aggctggagc ccagattgaa 540
ttccgagata tgctggaatc cacagccatc cactgggcat gtcgtggagg aaacctggat 600
gtcctgaaac tgttgctaaa caaaggtgcc aaaatcagtg cccgagacaa gctgctcagc 660
acagcgctgc acgtggcggt gaggaccggt cactacgagt gtgctgagca cctcatcgcc 720
tgcgaggcgg atctcaatgc caaggacaga gaaggagaca cccctctgca tgatgcggtg 780
aggttgaatc gctacaagat gatccggctc ttgatgacct tcggtgcgga cctcaatgtc 840
aagaactgtg ctgggaagac ccctatggat ctggtgttgc actggcagaa tggaaccaaa 900
gcgatattcg acagcctcaa ggagaatgcc tacaaaaact cgcgcatagc tacgttc 957
<210> 3
<211> 22
<212> DNA
<213> Artificial Sequence
<220>
<223> Primer
<400> 3
acatagacta acggctgcca ac 22
<210> 4
<211> 22
<212> DNA
<213> Artificial Sequence
<220>

<223> Primer

<400> 4

agctcctgtc gagtctcttt cc          22

<210> 5

<211> 22

<212> DNA

<213> Artificial Sequence

<220>

<223> Primer

<400> 5

gcagaatgga accaaagcga ta          22

<210> 6

<211> 23

<212> DNA

<213> Artificial Sequence

<220>

<223> Primer

<400> 6

aagtgaggac acaaaaggga gat          23

<210> 7

<211> 22

<212> DNA

<213> Artificial Sequence

<220>

<223> Primer

<400> 7

gaacgtagct atgcgcgagt tt          22

<210> 8

<211> 27

<212> DNA
<213> Artificial Sequence
<220>
<223> Primer
<400> 8
tcgacagcct caaggagaat gcctaca     27
<210> 9
<211> 19
<212> DNA
<213> Artificial Sequence
<220>
<223> Primer
<400> 9
tcttcctggc cttttggct     19
<210> 10
<211> 21
<212> DNA
<213> Artificial Sequence
<220>
<223> Primer
<400> 10
atctgtgttg gacaccgcac t     21
<210> 11
<211> 26
<212> DNA
<213> Artificial Sequence
<220>
<223> Primer
<400> 11

ccaggccata ttggagcaaa tcccgt 26
<210> 12
<211> 22
<212> DNA
<213> Artificial Sequence
<220>
<223> Primer
<400> 12
agcagaccca gatccaggag tt 22
<210> 13
<211> 22
<212> DNA
<213> Artificial Sequence
<220>
<223> Primer
<400> 13
tgtcaatgaa gccgtctctg tt 22
<210> 14
<211> 26
<212> DNA
<213> Artificial Sequence
<220>
<223> Primer
<400> 14
aaggaggcct tcacaatcat ggacca 26
<210> 15
<211> 21
<212> DNA
<213> Artificial Sequence

<220>

<223> Primer

<400> 15

atgacaactc ctcccgcttt g          21

<210> 16

<211> 25

<212> DNA

<213> Artificial Sequence

<220>

<223> Primer

<400> 16

ccagaaggta ggtctctatg tctgc          25

<210> 17

<211> 29

<212> DNA

<213> Artificial Sequence

<220>

<223> Primer

<400> 17

agttcatcag gatccacttt ggagccaca          29

<210> 18

<211> 22

<212> DNA

<213> Artificial Sequence

<220>

<223> Primer

<400> 18

tgtacgccaa caatgtcctg tc          22

<210> 19

<211> 22

<212> DNA

<213> Artificial Sequence

<220>

<223> Primer

<400> 19

cagtgcggta atttcctttt gc          22

<210> 20

<211> 26

<212> DNA

<213> Artificial Sequence

<220>

<223> Primer

<400> 20

aggcaccacc atgtaccctg gaattg          26

## Description

### TECHNICAL FIELD

The present invention relates to use of a disease-associated gene. More particularly, the present invention relates to a method of screening a drug using the disease-associated gene product, an antisense nucleotide to the disease-associated gene, which is useful as a diagnostic marker for heart diseases such as cardiomyopathy, myocardial infarction, heart failure, angina pectoris, etc., an antibody to the disease-associated gene product, and the like.

### BACKGROUND ART

Heart failure is considered to be myocardial contractile dysfunction. The following events are assumed to be a mechanism of developing heart failure. When the heart is unable to supply the volume of blood pumped by the heart sufficient to meet an increased demand from the body because of overloads including myocardial disorders, a mechanical abnormality of cardiac pump, a functional abnormality of cardiac pump, pressure overload and anemia caused by hypertension and pulmonary hypertension, acute nephritis, etc., the heart activates compensatory mechanisms such as the sympathetic nervous system, nerve-body fluid-endocrine system, etc. to attempt to maintain in vivo homeostasis. That is, to compensate for heart failure, 1) preload increases to incite an increased contractile force of the heart. Thus, sarcomeres lengthen and as a result, cardiac hypertrophy occurs, and 2) systolic unit of the heart muscle increases. As the result, myocardial hypertrophy is caused and 3) neurohumoral factors are activated to compensate for the state in which blood necessary for the whole body is not pumped, and myocardial fibrosis is advanced locally. This is fundamentally a mechanism to correct given overloads and adapt to them. It is yet understood that the mechanism is sometimes insufficiently activated and thus acts to advance heart failure; conversely, it is excessively activated to cause myocardial injury, which might deteriorate heart failure. As a result of activated compensatory mechanism, cardiomyocytes undergo hypertrophy leading to cardiac hypertrophy. However, when the injuries or overloads described above are chronically continued, the compensatory mechanism breaks down. That is, æ sufficient volume of blood is not supplied to hypertrophied cardiomyocytes to cause ischemia, which results in myocardial injury including cardiac contractility disorder, etc. to induce heart failure syndromes accompanied by reduced cardiac output, organ circulation damages, venostasis, retention of body fluid, etc. For treatment of these syndromes, it is required to improve damaged cardiomyocytes, strengthen heart protection, restore reduced cardiac function caused by myocardial contractility disorder and prevent the in vivo decompensation as its causes or improve excessive compensatory mechanism. Currently, for the treatment of these heart failure syndromes, there are clinically used cardiotonics including 1. cardiotonic glycosides such as digoxin, etc., 2. sympathomimetic agents such as dobutamine, etc., and 3. phosphodiesterase inhibitors such as amrinone, etc.; vasodilators such as hydralazine, calcium antagonists and angiotensin I converting enzyme inhibitors, angiotensin II receptor antagonists, etc. Also for the treatment of non-dilated cardiomyopathy, β blockers, etc. are employed. However, curative medicines which can prevent the excessive compensatory mechanism or prevent the decompensation (including apoptosis prevention) are unknown.

The present invention aims at developing a therapeutic drug from a standpoint of preventing the excessive compensatory mechanism or decompensation and intends to discover its survey target, thereby to find a gene with its increased expression in the heart on the development of heart failure and provide a method of screening a compound regulating the activity of a protein as its gene product, provide the compound obtainable by the screening method, and the like.

### DISCLOSURE OF INVENTION

The present inventors made extensive investigations to solve the problems described above and as a result, have found mRNA with its increased expression at the development of heart failure syndromes in model rat with myocardial infarction generated by coronary artery ligature.

Detailed investigations of the expression profile of said mRNA represented as cDNA in SEQ ID NO: 2 revealed that this mRNA increased on postoperative week 1 but decreased inversely on postoperative weeks 8 and prominently increased on postoperative weeks 20 and 30. From its base sequence, the mRNA was identified to be rat cardiac adriamycin responsive protein (hereinafter abbreviated as CARP, The Journal of Biological Chemistry (J. B. C.), 272, 22800-22808, 1997, Development, 124, 793-804, 1997). By the way, the 919th T of the sequence described in J. B. C., 272, 22800-22808, 1997 and of U50736 registered in NCBI (gene bank) is correctly C, and the CARP gene acquired by the inventors coincided with the sequence published in Development, 124, 793-804, 1997. Based on these findings, further investigations were continued and the present invention has come to be accomplished.

That is, the present invention provides the following features.
(1) A method of screening a compound that regulates the activity of a protein having the same or substantially the same amino acid sequence as the amino acid sequence represented by SEQ ID NO:1, or its partial peptide, or a salt thereof, which comprises using a protein having the same or substantially the same amino acid sequence as the amino acid sequence represented by SEQ ID NO:1, or its partial peptide, or a salt thereof;
(2) The screening method according to (1), wherein the protein having the same or substantially the same amino acid sequence as the amino acid sequence represented by SEQ ID NO:1, or its partial peptide, or a salt thereof, is expressed in the cytoplasm of a transformant transformed by a DNA containing a DNA encoding a protein having the same or substantially the same amino acid sequence as the amino acid sequence represented by SEQ ID NO:1, or its partial peptide, or a salt thereof;
(3) A method of screening a CARP expression promoting or inhibiting agent, which comprises assaying a level of CARP expressed, respectively, when a test compound is administered to a primary cardiomyocyte or myocardium-derived cell line (H9c2) capable of expressing CARP and when the test compound is not administered thereto;
(4) A kit for screening a compound that regulates the activity of a protein having the same or substantially the same amino acid sequence as the amino acid sequence represented by SEQ ID NO:1, or its partial peptide, or a salt thereof, comprising a protein having the same or substantially the same amino acid sequence as the amino acid sequence represented by SEQ ID NO:1, or its partial peptide, or a salt thereof;
(5) A compound or its salt that regulates the activity of a protein having the same or substantially the same amino acid sequence as the amino acid sequence represented by SEQ ID NO:1, or its partial peptide, or a salt thereof, which is obtainable by using the screening method according to (1) or the screening kit according to (4);
(6) A compound that suppresses the activity of a protein having the same or substantially the same amino acid sequence as the amino acid sequence represented by SEQ ID NO:1, or its partial peptide, or a salt thereof, which is obtainable by using the screening method according to (1) or the screening kit according to (3);
(7) A pharmaceutical comprising a compound or its salt that regulates the activity of a protein having the same or substantially the same amino acid sequence as the amino acid sequence represented by SEQ ID NO:1, or its partial peptide, or a salt thereof, which is obtainable by using the screening method according to (1) or the screening kit according to (4);
(8) The pharmaceutical according to (7), which is a preventive/therapeutic agent for a heat disease;
(9) A method of treating a heart disease which comprises administering the pharmaceutical according to (7) to a mammal;
(10) Use of the compound or its salt according to (6) to prepare a preventive/therapeutic agent for a heat disease;
(11) An antisense DNA having a base sequence complementary or substantially complementary to a DNA encoding a protein or its partial peptide having the same or substantially the same amino acid sequence as the amino acid sequence represented by SEQ ID NO:1;
(12) A pharmaceutical comprising the antisense DNA according to (11);
(13) A monoclonal antibody to a protein having the same or substantially the same amino acid sequence as the amino acid sequence represented by SEQ ID NO:1, or its partial peptide, or a salt thereof; and,
(14) A diagnostic agent or pharmaceutical comprising an antibody to a protein having the same or substantially the same amino acid sequence as the amino acid sequence represented by SEQ ID NO:1, or its partial peptide, or a salt thereof; and the like.
   The present invention further relates to the following features.
(15) The screening method according to (3), wherein the primary cardiomyocyte or myocardium-derived cell line (H9c2) is transformed by a DNA containing a DNA encoding a protein having the same or substantially the same amino acid sequence as the amino acid sequence represented by SEQ ID NO:1, or its partial peptide, or a salt thereof;
(16) The screening method according to (3), wherein the level of CARP expressed is assayed by northern blotting using a DNA encoding a protein having the same or substantially the same amino acid sequence as the amino acid sequence represented by SEQ ID NO:1, or its partial peptide, or a salt thereof;
(17) The screening method according to (3), wherein the level of CARP expressed is assayed by TaqMan PCR using a DNA encoding a protein having the same or substantially the same amino acid sequence as the amino acid sequence represented by SEQ ID NO:1, or its partial peptide, or a salt thereof;
(18) The screening method according to (3), wherein the level of CARP expressed is assayed using an anti-CARP monoclonal antibody;
(19) The screening method according to (3), wherein the primary cardiomyocyte or myocardium-derived cell line (H9c2) is incubated under lethal conditions;
(20) The screening method according to (19), wherein the lethal conditions are set to eliminate serum and a compound for reducing the expression level of CARP is screened;
(21) The screening method according to (19), wherein the lethal conditions are set to reduce a glucose level and a compound for reducing the expression level of CARP is screened;
(22) The screening method according to (19), wherein the lethal conditions are set to administer an antitumor agent having high toxicity to cardiomyocyte and a compound for increasing the expression level of CARP is screened;
(23) The screening method according to (22), wherein the antitumor agent having high toxicity to cardiomyocyte is adriamycin;
(24) The screening method according to (3), wherein a stimulation for stretching is given to the primary cardiomyocyte or myocardium-derived cell line (H9c2);
(25) The screening method according to (3), wherein endothelin or norepinephrine is administered to stimulate the primary cardiomyocyte or myocardium-derived cell line (H9c2) to cause hypertrophy;
(26) The screening method according to (3), wherein the expression level of CARP is assayed using as an indicator a respiration activating activity of the primary cardiomyocyte or myocardium-derived cell line (H9c2);
(27) The screening method according to (3), wherein the expression level of CARP is assayed using as an indicator a morphological change of the primary cardiomyocyte or myocardium-derived cell line (H9c2);
(28) The screening method according to (3), which is to screen a compound that maintains homeostasis of sarcomeric organization;
(29) A method of screening a CARP promoter activity-promoting or inhibiting agent, which comprises assaying an expression level or activity of reporter gene is assayed, respectively, when a test compound is administered to the primary cardiomyocyte or myocardium-derived cell line (H9c2) ligated with a gene encoding a reporter protein downstream a promoter sequence of the CARP gene and when not test compound is administered;
(30) The screening method according to (29), wherein the reporter protein is an enzyme;
(31) The screening method according to (30), wherein the enzyme is luciferase or beta-galactosidase;
(32) A method of screening 1) a compound having a cardiotonic activity or 2) a compound having heart protection against ischemia, which comprises measuring the cardiac function or coronary artery flow of the compound obtained in the screening method according to any one of (1) through (3);
(33) The screening method according to (32), wherein the cardiac function or coronary artery flow is assayed by using a Langendorf' perfusion heart;
(34) The screening method according to (32), wherein the cardiac function or coronary artery flow is assayed by using a model animal with myocardial infarction, a model with hypertension or a model with hypertrophy;
(35) A method of screening 1) a compound having a cardiotonic activity or 2) a compound having heart protection against ischemia, which comprises measuring ischemic tolerance to the compound obtained by the screening method according to any one of (1) through (3);
(36) The method according to (35), wherein ischemic tolerance is determined by measuring a size of infarction or cardiac function;
(37) A method of screening a compound having an activity of suppressing cardiac hypertrophy or an activity of potentiating compensatory cardiac hypertrophy, which comprises weighing the heart weight when a compound obtained by the screening method according to any one of (1) through (3) is administered to a non-human mammal; and,
(38) A method of screening a compound having an activity of improving mammalian QOL, which comprises determining the mortality rate or cardiovascular kinetic of a non-human mammal when a compound obtained by the screening method according to any one of (1) through (3) is administered to the non-human mammal; and the like.

### BRIEF DESCRIPTION OF THE DRAWINGS

FIG. 1 shows a tissue distribution of CARP gene in normal rat by northern blotting described in EXAMPLE 4, wherein numerals 1, 2, 3, 4, 5, 6, 7 and 8 denote heart, brain, spleen, lung, liver, skeletal muscle, kidney and testis, respectively.
FIG. 2 shows a tissue distribution of CARP gene in normal rat by dot blotting described in EXAMPLE 4.

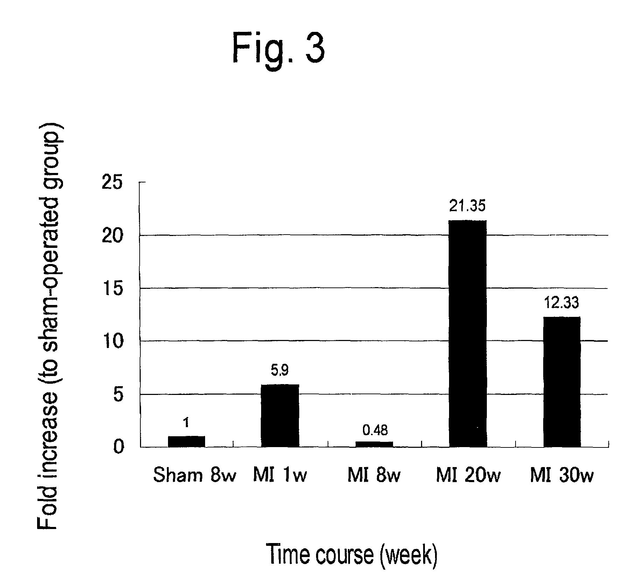

FIG. 3 shows a degree of variability obtained by dividing a change with passage of time in the CARP gene of a model rat with myocardial infarction described in EXAMPLE 5 by the measurement data of a sham-operated group, which is shown in terms of a fold increase. In the figure, the ordinate represents a fold increase obtained by dividing the number of copies (expression level) of the CARP gene in the left ventricle by the number of copies of a housekeeping gene or glycerol-3-phosphate dehydrogenase for correction and further dividing the corrected data by the measurement data of sham-operated group. Time course (week) on the abscissa shows the name of samples keeping track of models with heart failure. The symbol sham indicates the sham-operated group, and symbols MI 1w, MI 8w, MI 20w and MI 30w indicate the name of samples, respectively, when the heart was analyzed at postoperative 1, 8, 20 and 30 weeks.

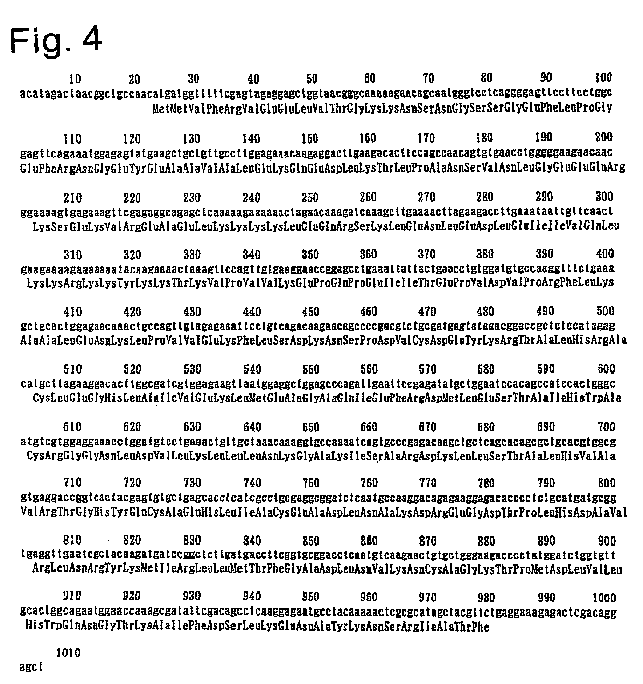

FIG. 4 shows the base sequence of rat CARP gene and its amino acid sequence encoding the gene.

The protein having the same or substantially the same amino acid sequence as the amino acid sequence represented by SEQ ID NO:1, which is employed in the present invention (hereinafter sometimes simply referred to as the protein of the present invention, the protein used in the present invention or CARP) may be any protein derived from human's and other warm-blooded animals' (e.g., guinea pig, rat, mouse, chiken, rabbit, swine, sheep, bovine, monkey, etc.) cells (e.g., hepatocyte, splenocyte, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocyte or interstitial cells; or the corresponding precursor cells, stem cells, cancer cells, etc.); or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; or, the protein may be a synthetic protein.

The amino acid sequence which has substantially the same amino acid sequence as the amino acid sequence represented by SEQ ID NO:1 includes an amino acid sequence having at least about 50% homology, preferably at least about 60% homology, more preferably at least about 70% homology, further more preferably at least about 80% homology, much more preferably at least about 90% homology, and most preferably at least about 95% homology, to the amino acid sequence represented by SEQ ID NO:1.

Preferred examples of the protein having substantially the same amino acid sequence as the amino acid sequence represented by SEQ ID NO: 1 are proteins having substantially the same amino acid sequence as the amino acid sequence represented by SEQ ID NO:1 and having an activity substantially equivalent to that of the protein having the amino acid sequence represented by SEQ ID NO:1, and the like.

The substantially equivalent activity includes, e.g., an activity of improving cardiac hypofunction, etc.

The term substantially equivalent is used to mean that such properties are equivalent in nature (for example, physiologically or pharmacologically). It is thus preferred that the cardiac hypofunction improving activity is equivalent (e.g., about 0.01 to about 100 times, preferably about 0.1 to about 10 times, more preferably 0.5 to about 2 times), and it is allowable that differences among grades such as the strength of these activities, molecular weight of the protein, etc. may even be present.

The activity such as the cardiac hypofunction improving activity, etc. may be assessed by echocardiograph (Cell, 97, 189-198, 1999) or by cardiac function through cardiac catheterization (Circulation Research, 69, 370-377, 1991). Furthermore, the activity may also be assessed by, e.g., activation of the renin-angiotensin system (RAS) such as angiotensin I converting enzyme (ACE), etc. using a commercially available kit (e.g., Peninsula Laboratories, Inc. or Phoenix Pharmaceuticals, Inc.), or by an increased activity of blood catecholamine (manufactured by TOSO Co., Ltd., full automatic catecholamine analyzer), etc. as an indicator.

Specific examples of the protein used in the present invention include so-called muteins such as proteins containing (i) an amino acid sequence represented by SEQ ID NO: 1, of which 1, 2 or more (preferably about 1 to about 30, more preferably about 1 to about 10, and most preferably several (1 to 5)) amino acids are deleted; (ii) an amino acid sequence represented by SEQ ID NO: 1, to which 1, 2 or more (preferably about 1 to about 30, more preferably about 1 to about 10, and most preferably several (1 to 5)) amino acids are added; (iii) an amino acid sequence represented by SEQ ID NO: 1, in which 1, 2 or more (preferably about 1 to about 30, more preferably about 1 to about 10, and most preferably several (1 to 5)) amino acids are inserted; (iv) an amino acid sequence represented by SEQ ID NO: 1, in which 1, 2 or more (preferably about 1 to about 30, more preferably about 1 to about 10, and most preferably several (1 to 5)) amino acids are substituted by other amino acids; or (v) a combination of the above amino acid sequences, and the like.

Throughout the specification, the proteins are represented in accordance with the conventional way of describing peptides, that is, the N-terminus (amino terminus) at the left hand and the C-terminus (carboxyl terminus) at the right hand. In the proteins of the present invention including the proteins containing the amino acid sequence shown by SEQ ID NO:1, the C-terminus is usually in the form of a carboxyl group (-COOH) or a carboxylate (-COO⁻) but may be in the form of an amide (-CONH₂) or an ester (-COOR).

Examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl group such as a phenyl-C₁₋₂ alkyl group (e.g., benzyl, phenethyl, etc.); or an α-naphthyl-C₁₋₂ alkyl group (e.g., α-naphthylmethyl, etc.); pivaloyloxymethyl, and the like.

Where the protein of the present invention contains a carboxyl group (or a carboxylate) at a position other than the C-terminus, it may be amidated or esterified and such an amide or ester is also included within the protein of the present invention. As the ester group, there may be used, e.g., the C-terminal ester, etc. described above.

Furthermore, examples of the protein used in the present invention include variants of the above protein, wherein the amino group at the N-terminal amino acid residue (e.g., methionine residue) is protected with a protecting group (e.g., a C₁₋₆ acyl group, e.g., formyl group, a C₁₋₆ alkanoyl group such as acetyl group, etc.); those wherein the N-terminal glutamine residue is cleaved in vivo and the glutamyl group thus formed is pyroglutaminated; those wherein substituents (e.g., -OH, -SH, amino group, imidazole group, indole group, guanidino group, etc.) on the side chain of amino acids in the molecule are protected with suitable protecting groups (e.g., a C₁₋₆ acyl group, e.g., formyl group, a C₁₋₆ alkanoyl group such as acetyl group, etc.), or conjugated proteins such as glycoproteins having sugar chains.

A specific example of the protein used in the present invention is, e.g., the rat-derived protein represented by SEQ ID NO: 1, or the like.

As the partial peptide of the protein used in the present invention, any partial peptide may be used, so long as it is a partial peptide of the aforesaid protein used in the present invention and has a property similar to the said protein used in the present invention.

As the partial peptide, there are employed, for example, peptides having the sequence of at least 20, preferably at least 50, more preferably at least 70, much more preferably 100 and most preferably at least 200 amino acids, in the amino acid sequence that constitutes the protein used in the present invention, etc.

The partial peptide used in the present invention may contains an amino acid sequence, of which 1, 2 or more (preferably about 1 to about 10, more preferably several (1 to 5)) amino acids are deleted; to which 1, 2 or more (preferably about 1 to about 20, more preferably about 1 to about 10, and most preferably several (1 to 5)) amino acids are added; in which 1, 2 or more (preferably about 1 to about 20, more preferably about 1 to about 10, and most preferably several (1 to 5)) amino acids are inserted; in which 1, 2 or more (preferably about 1 to about 10, more preferably several, and most preferably about 1 to about 5) amino acids are substituted by other amino acids.

In the partial peptide used in the present invention, the C-terminus may take any form of a carboxyl group (-COOH), a carboxylate (-COO⁻), an amide (-CONH₂) and an ester (-COOR).

As in the said proteins used in the present invention, the partial peptide used in the present invention further includes those in which the amino group of the N-terminal amino acid residue (e.g., methionine residue) is protected by a protecting group, those in which the N-terminal end is cleaved in vivo and the produced glutamine residue is pyroglutamated, those in which substituents on the side chains of amino acids are protected by appropriate protecting groups, or conjugated peptides having sugar chains bound thereto, namely, so-called glycopeptides, and the like.

The partial peptide used in the present invention can be also used as an antigen for producing an antibody.

As the salts of the protein or its partial peptides used in the present invention, there are salts with physiologically acceptable acids (e.g., inorganic acids or organic acids) or bases (e.g., alkali metal salts), with particular preference in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein, its partial peptides or salts thereof used in the present invention may be manufactured by a publicly known method used to purify a protein from human or other warm-blooded mammalian cells or tissues described above, or may also be manufactured by culturing a transformant containing a DNA encoding the protein. Furthermore, the protein, etc. may also be manufactured by modifications of protein synthesis, which will be described hereinafter.

Where the protein or the like is manufactured from human or mammalian tissues or cells, human or mammalian tissues or cells are homogenized and extracted with an acid or the like, and the extract is purified and isolated by a combination of chromatography techniques such as reversed phase chromatography, ion exchange chromatography, and the like.

To synthesize the protein, its partial peptide used in the present invention, or its salts or amides, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in the order of the sequences of the objective protein according to various condensation methods publicly known in the art. At the end of the reaction, the protein is excised from the resin and at the same time, the protecting groups are removed. Then, intramolecular disulfide bond-forming reaction is carried out in a highly diluted solution to obtain the objective protein or its partial peptides, or amides thereof.

For condensation of the protected amino acids described above, a variety of activation reagents for protein synthesis may be used, and carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein bond-forming reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group and ethoxycarbonyl group. Examples of such a group as suitable for use in the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein or partial peptide, for example, the α-carboxyl group of the carboxy terminal amino acid is first protected by amidation; the peptide (protein) chain is then extended from the amino group side to a desired length. Thereafter, a protein or its partial peptide in which only the protecting group of the N-terminal α-amino group has been eliminated and a protein or its partial peptide in which only the protecting group of the C-terminal carboxyl group has been eliminated are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedure similar to the preparation of the amidated protein or peptide above to give the desired esterified protein or peptide.

The partial peptide or salts thereof used in the present invention can be manufactured by publicly known methods for peptide synthesis, or by cleaving the protein of the present invention or a protein containing the protein of the present invention with an appropriate peptidase. For the peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in 1) - 5) below.
1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography, recrystallization, etc. to give the partial peptide used in the present invention. When the partial peptide obtained by the above methods is in a free form, they may be converted into an appropriate salt by a publicly known method; when they are obtained in a salt form, they may be converted into a free form or different salts by a publicly known method or its modification.

The DNA encoding the protein used in the present invention may be any one if it contains a base sequence encoding the protein used in the present invention. Also, the DNA may be any one of genomic DNA, genomic DNA library, cDNA derived from the cells or tissues described above, cDNA library derived from the cells or tissues described above and synthetic DNA.

The vector to be used for the library may be any of bacteriophage, plasmid, cosmid, phagemid and the like. In addition, the DNA can be directly amplified by reverse transcriptase polymerase chain reaction (hereinafter abbreviated as RT-PCR) with total RNA or mRNA fraction prepared from the above-described cells or tissues.

Specifically, the DNA encoding the protein used in the present invention may be any one of. for example, a DNA containing the base sequence represented by SEQ ID NO:2, or any DNA having a base sequence hybridizable to the base sequence represented by SEQ ID NO:2 under high stringent conditions and encoding a protein having an activity substantially equivalent to that of the protein used in the present invention.

Specific examples of the DNA that is hybridizable to the base sequence represented by SEQ ID NO:2 under high stringent conditions include a DNA having at least about 50% homology, preferably at least about 60% homology, more preferably at least about 70% homology, further more preferably at least about 80% homology, much more preferably at least about 90% homology, and most preferably at least about 95% homology, to the base sequence represented by SEQ ID NO:2.

The hybridization can be carried out by publicly known methods or by its modification, for example, according to the method described in Molecular Cloning, 2nd Ed., J. Sambrook et al., Cold Spring Harbor Lab. Press, 1989. A commercially available library may also be used according to the instructions of the attached manufacturer's protocol. The hybridization can be carried out preferably under high stringent conditions.

The high stringent conditions used herein are, for example, those in a sodium concentration at about 19 mM to about 40 mM, preferably about 19 mM to about 20 mM at a temperature of approximately 50 to 70°C, preferably approximately 60 to 65°C. In particular, hybridization conditions in a sodium concentration at about 19 mM at a temperature of about 65°C are most preferred.

More specifically, a DNA having the base sequence represented by SEQ ID NO: 2 may be used for the DNA encoding a protein having the amino acid sequence represented by SEQ ID NO: 1.

The DNA encoding the partial peptide used in the present invention may be any one if it contains a base sequence encoding the said partial peptide used in the present invention. Also, the DNA may be any one of genomic DNA, genomic DNA library, cDNA derived from the cells or tissues described above, cDNA library derived from the cells or tissues described above and synthetic DNA.

The DNA encoding the partial peptide used in the present invention may be any one of, for example, a DNA containing a part of DNA having the base sequence represented by SEQ ID NO:2, or any DNA having a base sequence hybridizable to the base sequence represented by SEQ ID NO:2 under high stringent conditions and containing a part of DNA encoding a protein having an activity substantially equivalent to that of the protein used in the present invention.

The DNA hybridizable to the base sequence represented by SEQ ID NO:2 has the same significance as described above.

For the method of hybridization and high stringent conditions, the same method and conditions as those described above apply.

For cloning of the DNA that completely encodes the protein or its partial peptide used in the present invention (hereinafter sometimes simply referred to as the protein of the present invention), the DNA may be either amplified by PCR using synthetic DNA primers containing a part of the base sequence of the protein of the present invention, or the DNA inserted into an appropriate vector can be screened by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989), etc. In the case of using commercially available library, the hybridization may be performed in accordance with the protocol described in the attached instructions.

Conversion of the base sequence of DNA can be carried out according to publicly known methods such as the ODA-LA PCR method, the gapped duplex method, the Kunkel method, etc. or modifications thereof, by using a publicly known kit available as Mutan™-super Express Km (Takara Shuzo Co., Ltd.), Mutan™-K (Takara Shuzo Co., Ltd.), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector of the protein of the present invention can be manufactured, for example, by (a) excising the desired DNA fragment from the DNA encoding the protein of the present invention, (b) followed by ligation of the DNA fragment with an appropriate expression vector downstream a promoter in the vector.

Examples of the vector include plasmids derived form *E. coli* (e.g., pBR322, pBR325, pUC12, pUC13), plasmids derived from *Bacillus subtilis* (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV (cytomegalovirus) promoter, SRα promoter or the like is preferably used. Where the host is bacteria of the genus *Escherichia,* preferred examples of the promoter include trp promoter, lac promoter, recA promoter, λP_{L} promoter, lpp promoter, T7 promoter, etc. In the case of using bacteria of the genus *Bacillus* as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Ampr), neomycin resistant gene (hereinafter sometimes abbreviated as Neor, G418 resistance), etc. In particular, when dhfr gene is used as the selection of a gene of interest marker together with dhfr gene-deficient Chinese hamster cells, selection of a gene of interest can also be made on thymidine free media.

If necessary, a signal sequence that matches with a host is added to the N-terminal side of the protein of the present invention. Examples of the signal sequence that can be used are Pho A signal sequence, OmpA signal sequence, etc. in the case of using bacteria of the genus *Escherichia* as the host; α-amylase signal sequence, subtilisin signal sequence, etc. in the case of using bacteria of the genus *Bacillus* as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing a DNA encoding the protein of the present invention thus constructed, transformants can be manufactured.

Examples of the host, which may be employed, are bacteria belonging to the genus *Escherichia*, bacteria belonging to the genus *Bacillus,* yeast, insect cells, insects, animal cells, and the like.

Specific examples of the bacteria belonging to the genus *Escherichia* include *Escherichia coli* K12 DHI [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus *Bacillus* include *Bacillus subtilis* MI114 [Gene, 24, 255 (1983)], 207-21 [Journal of Biochemistry, 95, 87 (1984)], etc.

Examples of yeast include *Saccharomyces cereviseae* AH22, AH22R⁻, NA87-11A, DKD-5D, 20B-12, *Schizosaccharomyces pombe* NCYC1913, NCYC2036, *Pichia pastoris* KM71, etc.

Examples of insect cells include, for the virus AcNPV, *Spodoptera frugiperda* cell (Sf cell), MG1 cell derived from mid-intestine of *Trichoplusia ni,* High Five™ cell derived from egg of *Trichoplusia ni,* cells derived from *Mamestra brassicae,* cells derived from *Estigmena acrea,* etc.; and for the virus BmNPV, established cell derived from silkworm (*Bombyx mori* N cell; BmN cell), etc. is used. Examples of the Sf cell which can be used are Sf9 cell (ATCC CRL1711) and Sf21 cell (both cells are described in Vaughn, J. L. et al., *In vivo,* 13, 213-217 (1977).

As the insect, for example, a larva of *Bombyx mori* can be used [Maeda et al., Nature, 315, 592 (1985)].

Examples of animal cells include monkey cell COS-7, Vero, Chinese hamster cell CHO (hereinafter referred to as CHO cell), dhfr gene deficient Chinese hamster cell CHO (hereinafter simply referred to as CHO(dhfr⁻) cell), mouse L cell, mouse AtT-20, mouse myeloma cell, rat GH 3, human FL cell, H9c2 cell, etc.

Bacteria belonging to the genus *Escherichia* can be transformed, for example, by the method described in Proc. Natl. Acad. Sci. U.S.A., 69, 2110 (1972), Gene, 17, 107 (1982), etc.

Bacteria belonging to the genus *Bacillus* can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by Shujunsha, or Virology, 52, 456 (1973).

Thus, the transformant transformed with the expression vector containing the DNA encoding the protein can be obtained.

Where the host is bacteria belonging to the genus *Escherichia* or the genus *Bacillus,* the transformant can be appropriately cultured in a liquid medium which contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for culturing the bacteria belonging to the genus *Escherichia* is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus *Escherichia* are used as the host, the transformant is usually cultivated at approximately 15 to 43°C for approximately 3 to 24 hours. If necessary, the culture may further be aerated or agitated.

Where the bacteria belonging to the genus *Bacillus* are used as the host, the transformant is cultivated generally at approximately 30 to 40°C for approximately 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to approximately 5 to 8. In general, the transformant is cultivated at approximately 20 to 35°C for approximately 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to approximately 6.2 to 6.4. Normally, the transformant is cultivated at about 27°C for approximately 3 to 5 days and, if necessary, the culture may be aerated or agitated.

Where animal cells are employed as the host, the transformant is cultivated in, for example, MEM medium containing approximately 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at approximately 30°C to 40°C for approximately 15 to 60 hours and, if necessary, the culture may be aerated or agitated.

As described above, the protein of the present invention can be produced in the cytoplasm of transformant or extracellularly.

The protein of the present invention can be separated and purified from the culture described above, e.g., by the following procedures.

When the protein of the present invention is extracted from the culture or cells, after cultivation, the transformants or cells are collected by a publicly known method and suspended in an appropriate buffer. The transformants or cells are then disrupted by publicly known methods such as ultrasonication, a treatment with lysozyme and/or freeze-thaw cycling, followed by centrifugation, filtration, etc. Thus, the crude extract of the protein can be obtained. The buffer used for the procedures may contain a protein modifier such as urea or guanidine hydrochloride, or a surfactant such as Triton X-100TM, etc. When the protein of the present invention is secreted in the culture broth, after completion of the cultivation, the supernatant can be separated from the transformants or cells to collect the supernatant by a publicly known method.

The supernatant or the protein of the present invention contained in the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, it can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein modifying enzyme so that the protein can be appropriately modified to partially remove a polypeptide. Examples of the protein-modifying enzyme include trypsin, chymotrypsin, arginyl endopeptidase, protein kinase, glycosidase and the like.

The protein of the present invention thus produced can be quantified by, for example, enzymeimmunoassay, Western blotting analysis, etc. using a specific antibody.

Antibodies to the protein, its partial peptide, or salts thereof used in the present invention may be any of polyclonal and monoclonal antibodies, so long as they can recognize the protein, its partial peptide, or salts thereof used in the present invention.

The antibodies to the protein, its partial peptide, or salts thereof used in the present invention (hereinafter sometimes merely referred to as the protein of the present invention) can be manufactured according to publicly known methods for producing antibodies or antisera, using the proteins of the present invention as antigens.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every 2 to 6 weeks and approximately 2 to 10 times in total. Examples of applicable warm-blooded animals are monkeys, rabbits, dogs, guinea pigs, mice, rats, sheep, goats and chicken with the use of mice and rats being preferred.

In the preparation of monoclonal antibody-producing cells, a warm-blooded animal, e.g., mice, immunized with an antigen wherein the antibody titer is noted is selected, then spleen or lymph node is collected after two to five days from the final immunization and antibody-producing cells contained therein are fused with myeloma cells from homozoic or heterozoic animal to give monoclonal. antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion promoting agent are polyethylene glycol (PEG), Sendai virus, etc., among which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by culturing at 20 to 40°C, preferably at 30 to 37°C for 1 to 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., a microplate) adsorbed with a protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, a method which comprises adding the supernatant of hybridoma to a solid phase adsorbed with an anti-immunoglobulin antibody or protein A, adding the protein labeled with a radioactive substance or an enzyme and detecting the monoclonal antibody bound to the solid phase, or the like.

The monoclonal antibody can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow there. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks, normally in 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out as in the separation and purification of polyclonal antibodies, following the procedures for separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, protein A or protein G, etc. and dissociating the binding to obtain the antibody].

### [Preparation of polyclonal antibody]

The polyclonal antibody of the present invention can be manufactured by publicly known methods or modifications thereof. For example, a complex of immunogen (protein antigen) and a carrier protein is formed and a mammal is immunized with the complex in a manner similar to the method described above for the manufacture of monoclonal antibody. The product containing the antibody to the protein of the present invention is collected from the immunized animal followed by separation and purification of the antibody.

In the complex of immunogen and carrier protein for immunizing mammal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin, keyhole limpet hemocyanin, etc. is coupled to hapten in a carrier-to-hapten weight ratio of approximately 0.1 to 20, preferably approximately 1 to 5.

A variety of condensation agents can be used for the coupling of carrier to hapten. Glutaraldehyde, carbodiimide, maleimide activated ester and activated ester reagents containing a thiol group, a dithiopyridyl group, etc. are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to about 6 weeks and about 3 to about 10 times in total.

The polyclonal antibody can be collected from the blood, ascites, etc. of warm-blooded animal immunized by the method described above, preferably from the blood.

The polyclonal antibody titer in antiserum can be assayed by the same procedure as used for the determination of serum antibody titer described above. The separation and purification of the polyclonal antibody can be carried out, following the method for the separation and purification of immunoglobulins performed as in the separation and purification of monoclonal antibodies described hereinabove.

The antisense nucleotide having a complementary or substantial complementary base sequence to the DNA encoding the protein or partial peptide used in the present invention (in the following description regarding the antisense nucleotide, these DNAs are hereinafter referred to as the DNA of the present invention) can be any antisense nucleotide, so long as it has a base sequence complementary or substantially complementary to the base sequence of the DNA of the present invention and capable of suppressing expression of the DNA, and preferably it is an antisense DNA.

The base sequence substantially complementary to the DNA of the present invention may, for example, be a base sequence having at least about 70% homology, preferably at least about 80% homology, more preferably at least about 90% homology and most preferably at least about 95% homology, to the full-length base sequence or partial base sequence of the base sequence complementary to the DNA of the present invention (i.e., complementary strand to the DNA of the present invention), and the like. In the entire base sequence of the complementary strand to the DNA of the present invention, an antisense nucleotide (e.g., an antisense DNA) having at least about 70% homology, preferably at least about 80% homology, more preferably at least about 90% homology and most preferably at least about 95% homology, to the complementary strand of the base sequence which encodes the N-terminal region in the protein of the present invention (e.g., the base sequence around the initiation codon, etc.).

The antisense nucleotide is generally constructed by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methylphosphonate, phosphorodithionate, etc. These antisense nucleotides may be synthesized using a publicly known DNA synthesizer, etc.

Hereinafter, the protein or partial peptide or salts thereof used in the present invention (hereinafter sometimes merely referred to as the protein of the present invention), the DNA encoding the protein or partial peptide of the present invention (hereinafter sometimes merely referred to as the DNA of the present invention), the antibody to the protein, partial peptide, or its salts of the present invention (hereinafter sometimes merely referred to as the antibody of the present invention) and the antisense nucleotide to the DNA of the present invention (hereinafter sometimes merely referred to as the antisense nucleotide of the present invention) are explained with respect to their utilities.

The protein of the present invention can be utilized as a disease marker, since its expression increases at the transitional state of myocardial infarction to heart failure. That is, the protein is useful as a marker for early diagnosis in post myocardial infarction heart failure or heart failure induced by other cardiac diseases (e.g., angina pectoris, cardiomyopathy, etc.), diagnosis of severity in conditions, or predication in progression of diseases.

Since post myocardial infarction heart failure or heart failure induced by other cardiac diseases (e.g., angina pectoris, cardiomyopathy, etc.) is prevented by administration of the antisense nucleotide (e.g., antisense DNA) of the gene encoding the protein of the present invention, the compound or its salt that regulates the activity of the protein of the present invention is considered to exhibit a similar activity.

Thus, a pharmaceutical composition comprising the antisense nucleotide (e.g., antisense DNA) of the gene encoding the protein of the present invention, the compound or its salt that regulates the activity of the protein of the present invention, or the antibody to the protein of the present invention can be used as therapeutic/preventive agent for, e.g., post myocardial infarction heart failure, or heart failure induced by other cardiac diseases (e.g., angina pectoris, cardiomyopathy, etc.).

### [1] Screening of drug candidate compound for disease

The protein of the present invention is increasingly expressed with cardiac hypofunction after myocardial infarction. Thus, the compound or its salt that regulates (inhibits or suppresses) the activity of the protein of the present invention can be used as a therapeutic/preventive agent for heart diseases such as post myocardial infarction heart failure (in particular, heart failure at the end stage of heart failure), etc. Also, reduced expression of the protein of the present invention is observed in the chronic stage of heart failure and thus, the compound or its salt that regulates (potentiates) the activity of the protein of the present invention can be used as a therapeutic/preventive agent for heart diseases such as post myocardial infarction heart failure (in particular, heart failure at the chronic stage of heart failure), etc.

Therefore, the protein of the present invention is useful as a reagent for screening the compound or its salts that regulates the activity of the protein of the present invention.

That is, the present invention provides:
(1) a method of screening a compound or its salt that regulates the activity of the protein of the present invention, e.g., a compound or its salt that regulates an activity of improving cardiac hypofunction caused by decompensation or an excessive compensation mechanism suppressing activity.

More specifically, the present invention provides, for example:
(2) a method of screening a compound or its salt that regulates the activity of the protein of the present invention, which comprises comparing (i) the case where a cell capable of producing the protein of the present invention is stretch-activated under low oxygen conditions and (ii) the case where a cell capable of producing the protein of the present invention and a test compound are stretch-activated under low oxygen conditions.

Specifically, in the screening method described above, the method is characterized by measuring, e.g., a gene expression level of the protein of the present invention in the cases (i) and (ii), and comparing them.

Herein, the low oxygen conditions described above are used to refer to conditions in an oxygen concentration, e.g., 20% O₂ or lower, e.g., 2% (NATURE, 394, 485-490, 1998). Stretch activation is applied by culturing myocyte on a deformable silicone substrate and stretching to apply a mechanical load (J. B. C., 271, 33592-33597, 1996; Circulation, 89, 2204-2211, 1994; J. B. C., 271, 3221-3228, 1996).

The present invention further provides:
(3) a method of screening a regulating agent which comprises comparing (iii) the case where a cell capable of producing the protein of the present invention or a cell bearing a cDNA encoding the protein of the present invention is cultured under lethal conditions, specifically when cultured under serum-free conditions or in the presence of an antitumor agent such as adriamycin, etc., having relatively strong toxicity against myocyte and (iv) the case where a mixture of a cell capable of producing the protein of the present invention or a cell bearing a cDNA encoding the protein of the present invention and a test compound is cultured under lethal conditions, specifically when cultured under serum-free conditions or in the presence of an antitumor agent such as adriamycin, etc., having relatively strong toxicity against myocyte.

In the screening method above, the method is characterized by measuring, e.g., a cell protecting activity and a gene expression level of the protein of the present invention in the cases (iii) and (iv), and comparing them.

The cell protecting activity can be expressed in terms of activation or survival rate of cardiomyocytes. Specifically, the cell protecting activity can be measured by the MTT assay, the trypan blue staining method or the Tunnel staining method (Cell, 97, 189-198, 1999), by which a generally accepted respiratory activity can be assayed. The CARP gene is believed to have a function of down-regulating heart-specific gene expression. It is thus considered that overexpression will damage cell functions. Conversely, excessively reduced expression induces undue cell activation so that cytotoxicity is believed to occur.

Furthermore, the present invention provides:
(4) a method of screening, which comprises regulating an activity of reporter gene in a reporter gene assay using a gene with its expression being regulated by the CARP gene product, for example, a promoter of an atrial natriuretic peptide (J. B. C., 272, 22800-22808, 1997; Development, 124, 798-804, 1997), etc. More specifically, the reporter gene assay is carried out by using as a host cell a primary cardiomyocyte or H9c2 cell line or a CARP gene-introduced primary cardiomyocyte or H9c2 cell line.

The reporter gene assay is carried out by constructing a plasmid ligated to a reporter gene such as β-galactosidase, chloramphenicol acetyltransferase, luciferase, etc. at the downstream of promoter region of, e.g., atrial natriuretic peptide gene (J. B. C., 272, 22800-22808, 1997; Development, 124, 798-804, 1997), and using a cell obtained by inserting the plasmid into a cardiomyocyte. In this cell, an enzyme activity of the reporter gene increases depending upon the expression level and activation of CARP protein, which is utilized for the screening system. Moreover, since the compound which is active in this system is a substance affecting the expression level and activation of the endogenous CARP gene, both of the CARP activity potentiator and the CARP activity inhibitor can be screened by this system.

A more preferred example of the screening method is a method which comprises quantifying the expression of CARP, respectively, when a test compound is administered to a primary cardiomyocyte or cardiomyocyte-derived cell line (H9c2) capable of expressing CARP and when no test compound is administered. Herein, the term "expression of CARP" is used to mean expression of the CARP gene or production of the CARP protein. As the primary cardiomyocyte or cardiomyocyte-derived cell line (H9c2) capable of expressing CARP, there are preferably employed a primary cardiomyocyte or a cardiomyocyte-derived cell line, or a primary cardiomyocyte or cardiomyocyte-derived cell line (H9c2), in which the CARP gene has been inserted, and a primary cardiomyocyte or cardiomyocyte-derived cell line (H9c2) transformed by the DNA of the present invention is preferably used.

The quantification can be performed by the northern blotting assay, the TaqMan PCR assay or, by inserting a plasmid ligated with a reporter gene such as luciferase, beta-galactosidase, etc. downstream the CARP promoter sequence (Development, 126, 4223-4234, 1999) into a primary cardiomyocyte or a cardiomyocyte-derived cell line and assaying the enzyme activity. Also, the expression of CARP can be quantified by using an anti-CARP antibody (preferably an anti-monoclonal antibody).

Where the expression is increased under the conditions for quantifying the expression, for example, set to eliminate serum or reduce a glucose level in a medium, or where the expression is increased by stimulating to induce hypertrophy such as a treatment with endothelin or norepinephrine, etc., such a compound that down-regulates the expression can be screened. On the other hand, where the expression of CARP gene is decreased by treating with an antitumor agent such as adriamycin, etc. having a relatively high toxicity against a cardiomyocyte, such a compound that suppresses the decrease in expression can be screened.

The thus obtained compound can be examined as follows.

When cells are damaged, e.g., by eliminating serum or reducing a glucose level in a medium or by stimulating to induce hypertrophy (endothelin or norepinephrine treatment, etc.) using primary cardiomyocytes or cardiomyocyte-derived cell lines or by treating these cells with an antitumor agent such as adriamycin having a relatively high toxicity against cardiomyocytes, the cell activation activity of test compound can be confirmed by assaying, e.g., by the MTT method, the activation activity such as a respiratory activity of the above cells in the cases wherein the test compound is administered and when no test compound is administered, and comparing the two cases in terms of the activity assayed.

Also, when cells are damaged as described above using primary cardiomyocytes or cardiomyocyte-derived cell lines (H9c2), the cells are morphologically observed (eosin-hematoxylin staining, actin immunostaining, etc.) when a test compound is administered and when no test compound is administered, whereby it can be confirmed that the compound maintains homeostasis of sarcomeric structure.

The compound obtained can be assessed by measuring the cardiac functions or coronary artery flow using the Langendorf's perfusion heart, if it is cardiotonic or heart protective against ischemia.

Further by administering the test compound to a model animal with myocardial infarction (coronary artery ligature, rat, mouse, dog, cat, swine, etc.), a model with hypertension (SHR, aorta ligature) or a model with hypertrophy (SHR, abdominal aorta ligature), pharmaceutical effects of the test compound can be assessed. These pharmaceutical effects can be examined by measuring a size of infarction or assaying the cardiac function by publicly known methods with regard to ischemic tolerance. Effects of the test compound for preventing hypertrophy or potentiating compensatory cardiac hypertrophy can be assessed by weighing the weight of heart before and after administration of the test compound and correcting with body weight. Furthermore, a QOL improving effect can be assessed by determining a mortality rate of the model animal described above or a non-human mammal (e.g., rat, mouse, dog, cat, swine, etc.) caused by administration of the test compound, or by measuring cardiac hemodynamics.

Examples of the test compound include peptides, proteins, non-peptide compounds of living body origin (sugars, lipids, etc.), synthetic compounds, fermentation products, cell extracts, plant extracts, animal tissue extracts, etc. These test compounds may be either novel or publicly known compounds.

To perform the screening method described above, cells capable of producing the protein of the present invention are cultured in a medium suitable for screening. Any medium is usable so far as it does not adversely affect expression of the gene encoding the protein of the present invention.

Examples of such cells capable of producing the protein of the present invention are primary cardiomyocytes having the ability of producing the protein of the present invention in nature, or hosts transformed by vectors bearing a DNA encoding the protein of the present invention (transformants). As the host, animal cells such as H9c2 cells, etc. are preferably employed.

The CARP gene-inserted primary cardiomyocytes (primary cardiomyocytes capable of producing the protein of the present invention) can be prepared according to the method described in, e.g., Japanese Patent Application No. 2000-194805, or Kiyota Goto and Hiroyuki Kaneko, Development of Method for Cardiovascular Studies (edited by Setsuro Ehashi), page 3, published by Gakkai Shuppan Center (1983).

For the screening, e,g., transformants with the protein of the present invention expressed intracellularly by culturing in accordance with the method described above are preferably employed.

The expression level of the gene of the present invention can be assayed by publicly known methods, e.g., northern blotting, reverse transcription-polymerase chain reaction (RT-PCR), TaqMan polymerase chain reaction, etc. or modifications thereof.

For example, when the gene expression level in the case (ii) described above is compared with that in the case (i) above, a test compound that inhibits (suppresses) or potentiates by at least about 20%, preferably at least about 30% and more preferably at least about 50% can be screened as the compound that inhibits (suppresses) or potentiates the activity of the protein of the present invention.

By administering the thus screened inhibitors (suppressors) at the end stage of heart failure in which increased expression of the CARP gene is noted, their cardiac function recovery effect can be expected. Also, potentiators are administered at the chronic stage of heart failure in which reduction in expression of the CARP gene is noted, whereby their heart protective effect due to protection of cardiomyocytes can be expected.

The screening kit of the present invention comprises the protein used in the present invention, its partial peptide or a salt thereof, or a cell capable of producing the protein used in the present invention or its partial peptide.

The compound or its salt, which is obtainable using the screening method or screening kit of the present invention, is a compound selected from the test compounds described above or salts thereof, for example, peptides, proteins, non-peptide compounds of living body origin (sugars, lipids, etc.), synthetic compounds, fermentation products, cell extracts, plant extracts, animal tissue extracts, plasma, etc., and are compounds that regulate (promote or inhibit (suppress)) the activity (an activity of promoting cardiac hypofunction) of the protein of the present invention.

As salts of these compounds, salts similar to those of the protein of the present invention described above are employed.

The compound or its salt that regulates (promote or inhibit (suppress)) the activity of the protein of the present invention is useful as a drug such as a therapeutic/preventive agent for, e.g., diseases characterized by cardiac hypofunction (heart diseases) such as post myocardial infarction heart failure or angina pectoris, cardiomyopathy, and heart failure induced by diseases such as angina pectoris, cardiomyopathy, etc.

When the compound or its salt, which is obtainable using the screening method or screening kit of the present invention, is used as the therapeutic/preventive agent described above, it may be prepared into pharmaceutical preparations in a conventional manner, for example, in the form of tablets, capsules, elixirs, microcapsules, a sterile solution, a suspension, etc.

Since the thus obtained pharmaceutical preparation is safe and low toxic, the preparation can be administered orally or parenterally to human or other warm-blooded animal (e.g., mouse, rat, rabbit, sheep, swine, bovine, horse, chicken, cat, dog, monkey, chimpanzee, etc.).

The dose of the compound or its salt varies depending on activity, target disease, subject to be administered, route for administration, etc.; when the compound or its salt that regulates the activity of the protein of the present invention is orally administered for the treatment of, e.g., heart failure, the dose is normally about 0.1 to about 100 mg, preferably about 1.0 to about 50 mg, and more preferably about 1.0 to about 20 mg per day for adult (as 60 kg body weight). In parenteral administration, the single dose of the compound or its salt varies depending on subject to be administered, target disease, etc., but when the compound or its salt that regulates the activity of the protein of the present invention is normally administered to adult (as 60 kg body weight) for the treatment of, e.g., heart failure in the form of injection, it is advantageous to administer the compound or its salt intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

### [2] Quantification of the protein of the present invention or its partial peptide, or salts thereof

The antibody to the protein of the present invention (hereinafter sometimes merely referred to as the antibody of the present invention) is capable of specifically recognizing the protein of the present invention and thus, can be used for quantification of the protein of the present invention in a test fluid, in particular, for quantification by sandwich immunoassay.

That is, the present invention provides:
(i) a method for quantification of the protein of the present invention in a test fluid, which comprises competitively reacting the antibody of the present invention with a test fluid and a labeled form of the protein of the present invention, and measuring a ratio of the labeled protein of the present invention bound to said antibody; and,
(ii) a method for quantification of the protein of the present invention in a test fluid, which comprises simultaneously or continuously reacting the test fluid with the antibody of the present invention immobilized on an insoluble carrier and a labeled form of the another antibody of the present invention, and measuring the activity of labeling agent on the immobilized carrier.

In the method (ii) described above, it is preferred that one antibody is capable of recognizing the N-terminal region of the protein of the present invention, while another antibody is capable of reacting with the C-terminal region of the protein of the present invention.

The monoclonal antibody to the protein of the present invention (hereinafter sometimes referred to as the monoclonal antibody of the present invention) may be used to quantify the protein of the present invention. In addition, the protein of the present invention can also be detected by means of tissue staining, etc. For these purposes, the antibody molecule *per se* may be used, or F(ab')₂, Fab' or Fab fractions of the antibody molecule may be used as well.

The method for quantification using the antibody of the present invention is not particularly limited, and any method may be used so far as it relates to a method, in which the amount of an antibody, antigen or antibody-antigen complex can be detected by a chemical or a physical means, depending on or corresponding to the amount of antigen (e.g., the amount of the protein) in a test fluid to be assayed, and then calculated using a standard curve prepared by a standard solution containing the known amount of antigen. Advantageously used are, for example, nephrometry, competitive method, immunometric method and sandwich method; in view of sensitivity and specificity, the sandwich method, which will be described later, is particularly preferred.

Examples of labeling agents, which are used for the assay method using the same, are radioisotopes, enzymes, fluorescent substances, luminescent substances, etc. Examples of radioisotopes are [¹²⁵I], [¹³¹I], [³H], [¹⁴C], etc. Preferred examples of enzymes are those that are stable and have a high specific activity, which include β-galactosidase, β-glucosidase, alkaline phosphatase, peroxidase, malate dehydrogenase, etc. Examples of fluorescent substances are fluorescamine, fluorescein isothiocyanate, etc. Examples of luminescent substances are luminol, a luminol derivative, luciferin, lucigenin, etc. Furthermore, a biotin-avidin system may be used as well for binding an antibody or antigen to a labeling agent.

In the insolubilization of antigens or antibodies, physical adsorption may be used. Alternatively, chemical binding that is conventionally used for insolubilization or immobilization of proteins, enzymes, etc. may be used as well. Examples of the carrier include insoluble polysaccharides such as agarose, dextran, cellulose, etc.; synthetic resins such as polystyrene, polyacrylamide, silicone, etc.; or glass; and the like.

In the sandwich method, a test fluid is reacted with an insolubilized form of the monoclonal antibody of the present invention (primary reaction), then reacted with a labeled form of the monoclonal antibody of the present invention (secondary reaction) and the activity of the labeling agent on the insoluble carrier is assayed; thus, the amount of the protein of the present invention in the test fluid can be determined. The primary and secondary reactions may be carried out in a reversed order, simultaneously or sequentially with intervals. The type of the labeling agent and the method of insolubilization may be the same as those described hereinabove. In the immunoassay by the sandwich method, it is not always necessary that the antibody used for the labeled antibody and for the solid phase should be one type or one species but a mixture of two or more antibodies may also be used for the purpose of improving the assay sensitivity, etc.

In the method of assaying the protein of the present invention by the sandwich method according to the present invention, preferred monoclonal antibodies of the present invention used for the primary and the secondary reactions are antibodies, which binding sites to the protein of the present invention are different from each other. Thus, the antibodies used in the primary and secondary reactions are those wherein, when the antibody used in the secondary reaction recognizes the C-terminal region of the protein, the antibody recognizing the site other than the C-terminal regions, e.g., recognizing the N-terminal region, is preferably used in the primary reaction.

The monoclonal antibody of the present invention may be used in an assay system other than the sandwich method, such as the competitive method, the immunometric method or the nephrometry.

In the competitive method, an antigen in a test fluid and a labeled antigen are competitively reacted with an antibody, then an unreacted labeled antigen (F) and a labeled antigen bound to the antibody (B) are separated (i.e., B/F separation) and the labeled amount of either B or F is measured to determine the amount of the antigen in the test fluid. In the reactions for such a method, there are a liquid phase method in which a soluble antibody is used as the antibody and the B/F separation is effected by polyethylene glycol, while a second antibody to the antibody is used, and a solid phase method in which an immobilized antibody is used as the first antibody or a soluble antibody is used as the first antibody, while an immobilized antibody is used as the second antibody.

In the immunometric method, an antigen in a test fluid and an immobilized antigen are competitively reacted with a given amount of a labeled antibody followed by separating the solid phase from the liquid phase; or an antigen in a test fluid and an excess amount of labeled antibody are reacted, then an immobilized antigen is added to bind an unreacted labeled antibody to the solid phase and the solid phase is separated from the liquid phase. Thereafter, the labeled amount of any of the phases is measured to determine the antigen amount in the test fluid.

In the nephrometry, the amount of insoluble sediment, which is produced as a result of the antigen-antibody reaction in a gel or in a solution, is measured. Even when the amount of an antigen in a test fluid is small and only a small amount of the sediment is obtained, a laser nephrometry utilizing laser scattering can be suitably used.

In applying each of those immunoassays to the assay method of the present invention, any special conditions, operations, etc. are not required. The assay system for the protein of the present invention may be constructed in addition to conditions or operations conventionally used for each of the methods, taking technical consideration by one skilled in the art into account. For the details of such conventional technical means, a variety of reviews, reference books, etc. may be referred to.

For example, reference is made to Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immuochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (published by Academic Press); etc.

As described above, the protein of the present invention can be quantified with high sensitivity, using the antibody of the present invention.

In addition, by quantifying a level of the protein of the present invention using the antibody of the present invention, (1) when an increased level of the protein of the present invention is detected, it can be diagnosed that one suffers from a disease characterized by cardiac hypofunction such as post myocardial infarction heart failure or angina pectoris, cardiomyopathy, and a disease including heart failure, etc. induced by a heart disease such as angina pectoris, cardiomyopathy, etc., or it is highly likely to suffer from such a disease in the future.

The antibody of the present invention can be employed to specifically detect the protein of the present invention present in a test fluid such as a body fluid, tissues, etc. The antibody may also be used for the preparation of an antibody column used to purify the protein of the present invention, detect the protein of the present invention in each fraction upon purification, analysis of the behavior of the protein of the present invention in a cell under investigation.

### [3] Gene diagnostic agent

Using the DNA of the present invention as a probe, an abnormality of the DNA or mRNA (gene abnormality) encoding the protein of the present invention or the partial peptide thereof in human or other warm-blooded animal (e.g., rat, mouse, guinea pig, rabbit, chicken, sheep, swine, bovine, horse, cat, dog, monkey, chimpanzee, etc.) can be detected. Thus, the DNA of the present invention is useful as a gene diagnostic agent for damages to the DNA or mRNA, its mutation or its decreased expression, or increased expression or overexpression of the DNA or mRNA, or the like.

The gene diagnosis using the DNA of the present invention described above can be performed by, for example, publicly known northern hybridization assay or PCR-SSCP assay (Genomics, 5, 874-879 (1989); Proceedings of the National Academy of Sciences of the United States of America, 86, 2766-2770 (1989)), etc.

When overexpression is detected by northern hybridization or when mutation of DNA is detected by PCR-SSCP, it can be diagnosed that it is highly likely to suffer from a disease such as heart disease accompanying cardiac hypofunction.

### [4] Pharmaceutical comprising antisense nucleotide

The antisense nucleotide of the present invention that binds complementarily to the DNA of the present invention to suppress expression of the DNA (e.g., antisense DNA) is low toxic and can regulate (inhibit or suppress) the function (e.g., a cardiac hypofunction promoting activity, etc.) of the protein of the present invention or the DNA of the present invention *in vivo.* Thus, the antisense nucleotide can be used as an agent for the treatment/prevention of, e.g., heart disease accompanied by cardiac hypofunction.

When the antisense nucleotide described above is used as the therapeutic/prophylactic agent described above, the antisense nucleotide can be prepared into pharmaceutical preparations by publicly known methods, and then administered.

For example, when the antisense nucleotide (e.g., antisense DNA) is used, the antisense nucleotide (e.g., antisense DNA) alone is administered directly, or the antisense nucleotide is inserted into an appropriate vector such as retrovirus vector, adenovirus vector, adenovirus-associated virus vector, etc., and administered orally or parenterally to human or mammal (e.g., rat, rabbit, sheep, swine, bovine, cat, dog, monkey, etc.) in a conventional manner. The antisense nucleotide may be administered in its intact form, or with a physiologically acceptable carrier to assist its uptake by gene gun or through a catheter such as a hydrogel catheter.

The dose of the antisense nucleotide (e.g., antisense DNA) varies depending on target disease, subject to be administered, route for administration, etc.; for example, when the antisense nucleotide (e.g., antisense DNA) of the present invention is orally administered for the treatment of, e.g., heart failure, the antisense nucleotide (e.g., antisense DNA) is normally administered to adult (60 kg body weight) in a dose of about 0.1 to about 100 mg per day.

In addition, the antisense nucleotide (e.g., antisense DNA) may also be employed as an oligonucleotide probe for diagnosis to examine the presence of the DNA of the present invention in tissues or cells and states of its expression.

### [5] Pharmaceuticals comprising the antibody of the present invention

The antibody of the present invention having an activity of neutralizing the activity of the protein of the present invention can be used as a preventive/therapeutic agent for heart diseases characterized by cardiac hypofunction such as post myocardial infarction heart failure or angina pectoris, cardiomyopathy, and heart failure, etc. induced by diseases such as angina pectoris, cardiomyopathy, etc.

The preventive/therapeutic agent described above comprising the antibody of the present invention for the diseases described above is low toxic, and can be administered orally or parenterally to human or mammal (e.g., rat, rabbit, sheep, swine, bovine, cat, dog, monkey, etc.), in its liquid form as it stands, or as a pharmaceutical composition in a suitable preparation form. The dose varies depending on subject to be administered, target disease, condition, route for administration, etc.; when the pharmaceutical is administered to adult for the treatment/prevention of, e.g., heart failure, it is generally advantageous to administer the antibody of the present invention intravenously about 1 to about 5 times a day, preferably about 1 to 3 times a day, in a single dose of about 0.01 to about 20 mg/kg body weight, preferably about 0.1 to about 10 mg/kg body weight, and more preferably about 0.1 to about 5 mg/kg body weight. For other parenteral administration and oral administration, the dose corresponding to the dose above can be administered; when the condition is especially severe, the dose may be increased accordingly to the condition.

The antibody of the present invention may be administered in itself or as an appropriate pharmaceutical composition. The pharmaceutical composition used for the administration described above contains a pharmacologically acceptable carrier with the aforesaid compounds or salts thereof, a diluent or excipient. Such a composition is provided in a pharmaceutical preparation suitable for oral or parenteral administration.

That is, examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration are injections, suppositories, etc. and the injections include the form of intravenous, subcutaneous, transcutaneous, intramuscular and drip injections, etc. Such injections are prepared by publicly known methods, e.g., by dissolving, suspending or emulsifying the aforesaid antibody or its salts in a sterile aqueous or oily liquid medium. For the aqueous medium for injection, for example, physiological saline and isotonic solutions containing glucose and other adjuvant, etc. are used. Appropriate dissolution aids, for example, alcohol (e.g., ethanol), polyalcohol (e.g., propylene glycol, polyethylene glycol), nonionic surfactant [e.g., polysorbate 80, HCO-50 (polyoxyethylene (50 mols) adduct of hydrogenated castor oil)] may be used in combination. For the oily solution, for example, sesame oil, soybean oil and the like are used, and dissolution aids such as benzyl benzoate and benzyl alcohol may be used in combination. The thus prepared liquid for injection is normally filled in an appropriate ampoule. The suppository used for rectal administration is prepared by mixing the aforesaid antibody or its salts with conventional suppository base.

The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the antibody described above is contained generally in a dose of 5 to 500 mg per unit dosage form, 5 to 100 mg especially for injections and 10 to 250 mg for other dosage forms.

Each composition described above may further contain other active components unless formulation with the aforesaid antibody causes any adverse interaction.

### [6] DNA transgenic animal

The present invention provides a non-human mammal bearing an exogenous DNA encoding the protein, etc. of the present invention (hereinafter referred to as the exogenous DNA of the present invention) or its mutant DNA (sometimes simply referred to as the exogenous mutant DNA of the present invention).

Thus, the present invention provides:
(1) a non-human mammal bearing the exogenous DNA or its mutant DNA;
(2) the mammal according to (1), wherein the non-human mammal is a rodent;
(3) the mammal according to (2), wherein the rodent is mouse or rat; and,
(4) a recombinant vector bearing the exogenous DNA of the present invention or its mutant DNA and capable of expressing in a mammal.

The non-human mammal bearing the exogenous DNA of the present invention or its mutant DNA (hereinafter simply referred to as the DNA transgenic animal of the present invention) can be prepared by transfecting a desired DNA to an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method etc. Also, it is possible to transfect the exogenous DNA of the present invention to a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to create the transgenic animal of the present invention.

Examples of the non-human mammal that can be used include bovine, swine, sheep, goats, rabbits, dogs, cats, guinea pigs, hamsters, mice, rats, and the like. Above all, preferred are rodents, especially mice (e.g., C57BL/6 strain, DBA2 strain, etc. for a pure line and for a cross line, B6C3F₁ strain, BDF₁ strain B6D2F₁ strain, BALB/c strain, ICR strain, etc.) or rats (e.g., Wistar, SD, etc.), since they are relatively short in ontogeny and life cycle from a standpoint of creating model animals for disease.

"Mammals" in a recombinant vector that can be expressed in mammals include, in addition to the aforesaid non-human mammals, human, etc.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of original DNA of the present invention, specifically DNAs resulting from base addition, deletion, substitution with other bases, etc. and further including abnormal DNA.

The abnormal DNA is intended to mean the DNA that expresses the abnormal protein of the present invention and exemplified by such a DNA that expresses a protein capable of suppressing the functions of the normal protein of the present invention.

The exogenous DNA of the present invention may be any one of those derived from a mammal of the same species as, or a different species from, the mammal as the target animal. In transfecting the DNA of the present invention, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the protein of the present invention, there are *Escherichia coli*-derived plasmids, *Bacillus subtilis*-derived plasmids, yeast-derived plasmids, bacteriophages such as λ phage, retroviruses such as Moloney leukemia virus, etc., and animal viruses such as vaccinia virus, baculovirus, etc. Of these vectors, *Escherichia coli*-derived plasmids, *Bacillus subtilis*-derived plasmids, or yeast-derived plasmids, etc. are preferably used.

Examples of these promoters for regulating the DNA expression include 1) promoters for DNA derived from viruses (e.g., simian virus, cytomegalovirus, Moloney leukemia virus, JC virus, breast cancer virus, poliovirus, etc.), and 2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle *α* actin, preproencephalin A, vasopressin, etc. Among them, cytomegalovirus promoters, human polypeptide chain elongation factor 1α (EF-1α) promoters, human and chicken β actin promoters etc., which are capable of highly expressing in the whole body, are preferred.

It is preferred that the vectors described above have a sequence for terminating the transcription of the desired messenger RNA in the DNA transgenic animal (generally termed terminator); for example, a sequence of each DNA derived from viruses and various mammals. SV40 terminator of the simian virus or the like is preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be acquired using as a starting material the entire genomic DNA or its portion of liver, kidney, thyroid cell or fibroblast origin from various mammals (e.g., human, rabbits, dogs, cats, guinea pigs, hamsters, rats, mice, etc.) or of various commercially available genomic DNA libraries, or using complementary DNA prepared by a publicly known method from RNA of liver, kidney, thyroid cell or fibroblast origin as a starting material. In addition, an exogenous abnormal DNA can be prepared as a DNA having a translational region wherein the translational region of the normal protein from the above-mentioned cells or tissues is mutated by the site-directed mutagenesis.

The translational region can be prepared by a conventional DNA engineering technique, in which the DNA is ligated downstream the aforesaid promoter and if desired, upstream the translation termination site, as a DNA construct capable of expressing in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by mating.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the exogenous DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

By acquiring a homozygotic animal having the transfected DNA in both of homologous chromosomes and mating a male and female of the animal, all offspring can be passaged to excessively have the DNA.

In a non-human mammal bearing the normal DNA of the present invention, the normal DNA of the present invention is expressed to a high level, and may eventually develop the hyperfunction of the protein of the present invention by promoting the functions of endogenous normal DNA. Therefore, the animal can be utilized as a pathologic model animal for such a disease. Specifically, using the normal DNA transgenic animal of the present invention, it becomes possible to elucidate the hyperfunction by the protein of the present invention and to clarify the pathological mechanism of the disease associated with the protein of the present invention and to determine how to treat the disease.

Furthermore, since a mammal transfected with the exogenous normal DNA of the present invention exhibits an increasing symptom of the protein of the present invention librated, the animal is usable for a test of screening a therapeutic agent for disease associated with the protein of the present invention.

On the other hand, non-human mammal having the exogenous abnormal DNA of the present invention can be passaged under normal breeding conditions as the DNA-bearing animal by confirming the stable retaining of the exogenous DNA via crossing. In addition, the objective exogenous DNA can be utilized as a starting material by inserting the DNA into the plasmid described above. The DNA construct with a promoter can be prepared by using conventional DNA engineering techniques. Transfection of the abnormal DNA of the present invention at the fertilized egg cell stage is preserved to be present in all of the germinal and somatic cells of the mammals to be targeted. The fact that the abnormal DNA of the present invention is present in the germinal cells of the target animal after DNA transfection means that all of the offspring of the prepared animal have the abnormal DNA of the present invention in all of the germinal and somatic cells. Such an offspring that passaged the exogenous DNA of the present invention contains the abnormal DNA of the present invention in all of the germinal and somatic cells. A homozygous animal having the introduced DNA on both of homologous chromosomes can be acquired and then by mating these male and female animals, all the offspring can be bred to have the DNA.

Since the non-human mammal having the abnormal DNA of the present invention expresses the abnormal DNA of the present invention at a high level, the animal may cause the function inactive type inadaptability of the protein of the present invention by inhibiting the functions of the endogenous normal DNA, and can be utilized as its disease model animal. For example, using the abnormal DNA-transfected animal of the present invention, it is possible to elucidate the mechanism of the function inactive type inadaptability of the protein of the present invention and to study a method for treatment of this disease.

More specifically, the transgenic animal of the present invention expressing the abnormal DNA of the present invention to a high level is also expected to serve as an experimental model for the elucidation of the mechanism of the functional inhibition (dominant negative effect) of a normal protein by the abnormal protein of the present invention in the function inactive type inadaptability of the protein of the present invention.

A mammal bearing the transfected abnormal exogenous DNA of the present invention is also expected to serve to screen a candidate drug for the treatment of the function inactive type inadaptability of the protein of the present invention, since the protein of the present invention is increased in such an animal in its free form.

Other potential applications of two kinds of the transgenic animals described above include:
1) use as a cell source for tissue culture;
2) analysis of relevance to a protein that is specifically expressed or activated by the protein of the present invention, through direct analysis of the DNA or RNA in tissues of the DNA transgenic animal of the present invention or by analysis of the protein tissue expressed by the DNA;
3) research in the function of cells derived from tissues that are cultured usually only with difficulty, using cells of tissues bearing the DNA cultured by a standard tissue culture technique;
4) screening of a drug that enhances the function of cells using the cells described in 3) above; and,
5) isolation and purification of the variant protein of the present invention and preparation of an antibody thereto.

Furthermore, clinical conditions of a disease associated with the protein of the present invention, including the function inactive type inadaptability of the protein of the protein of the present invention can be determined using the DNA transgenic animal of the present invention. Also, pathological findings on each organ in a disease model associated with the protein of the present invention can be obtained in more detail, leading to development of a new method for treatment as well as the research and therapy of any secondary disease associated with the disease.

It is also possible to acquire a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve as identification of cells capable of producing the protein of the present invention, and as studies on association with apoptosis, differentiation or propagation or on the mechanism of signal transduction in these properties to inspect any abnormality therein. Thus, the DNA transgenic animal can provide an effective research material for the protein of the present invention and for elucidating the function and effect thereof.

To develop pharmaceuticals for the treatment of diseases associated with the protein of the present invention, including the function inactive type inadaptability of the protein of the present invention, using the DNA transgenic animal of the present invention, an effective and rapid method for screening can be provided by using the method for inspection and the method for quantification, etc. described above. It is also possible to investigate and develop a method for DNA therapy for the purpose of treating diseases associated with the protein of the present invention, using the DNA transgenic animal of the present invention or a vector capable of expressing the exogenous DNA of the present invention.

### [7] Knockout animal

The present invention provides a non-human mammal embryonic stem cell bearing the DNA of the present invention that is inactivated and a non-human mammal deficient in expressing the DNA of the present invention.

Thus, the present invention provides:
(1) a non-human embryonic stem cell in which the DNA of the present invention is inactivated;
(2) the embryonic stem cell according to (1), wherein the DNA is inactivated by introducing a reporter gene (e.g., β-galactosidase gene derived from *Escherichia coli*);
(3) the embryonic stem cell according to (1), which is resistant to neomycin;
(4) the embryonic stem cell according to (1), wherein the non-human mammal is a rodent;
(5) an embryonic stem cell according to (4), wherein the rodent is mouse;
(6) a non-human mammal deficient in expressing the DNA of the present invention, wherein the DNA of the present invention is inactivated;
(7) the non-human mammal according to (6), wherein the DNA is inactivated by inserting a reporter gene (e.g., β-galactosidase gene derived from *Escherichia coli*) therein and the reporter gene is capable of being expressed under control of a promoter for the DNA of the present invention;
(8) the non-human mammal according to (6), which is a rodent;
(9) the non-human mammal according to (8), wherein the rodent is mouse; and,
(10) a method for screening a compound or its salt that promotes or inhibits the promoter activity for the DNA of the present invention, which comprises administering a test compound to the mammal of (7) and detecting expression of the reporter gene.

The non-human mammal embryonic stem cell, in which the DNA of the present invention is inactivated, refers to a non-human mammal embryonic stem cell that suppresses the ability of the non-human mammal to express the DNA by artificially mutating the DNA of the present invention, or that the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell, in which the DNA of the present invention is inactivated (hereinafter merely referred toas the ES cell with the DNA of the present invention inactivated or the knockout ES cell of the present invention), can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the function of exon, or integrating to a chromosome of the subject animal by, e.g., homologous recombination, a DNA sequence which terminates gene transcription (e.g., polyA additional signal, etc.) in the intron between exons to, thus inhibit the synthesis of complete messenger RNA and eventually destroy the gene (hereinafter simply referred to as targeting vector). The thus-obtained ES cells to the Southern hybridization analysis with a DNA sequence on or near the DNA of the present invention as a probe, or to PCR analysis with a DNA sequence on the targeting vector and another DNA sequence near the DNA of the present invention, which is not included in the targeting vector as primers, to screen the knockout ES cell of the present invention.

The parent ES cells to inactivate the DNA of the present invention by homologous recombination, etc. may be of a strain already established as described above, or may be originally established in accordance with a modification of the known method by Evans and Kaufman *supra.* For example, in the case of mouse ES cells, currently it is common practice to use ES cells of the 129 strain. However, since their immunological background is obscure, the C57BL/6 mouse or the BDF₁ mouse (F₁ hybrid between C57BL/6 and DBA/2), wherein the low ovum availability in the C57BL/6 mouse has been improved by crossing with DBA/2, may be preferably used, instead of obtaining a pure line of ES cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are used in general. In addition, embryos are preferably collected at the 8-cell stage, and after culturing until the blastocyte stage, the embryos are used to efficiently obtain a large number of early stage embryos.

Although the ES cells used may be of either sex, male ES cells are generally more convenient for generation of a germ cell line chimera and are therefore preferred. It is desirable to identify sexes as soon as possible also in order to save painstaking culture time.

Methods for sex identification of the ES cell include the method, in which a gene in the sex-determining region on the Y-chromosome is amplified by the PCR process and detected. When this method is used, one colony of ES cells (about 50 cells) is sufficient for sex-determination analysis, which karyotype analysis requires about 10⁶ cells; therefore, the first selection of ES cells at the early stage of culture can be based on sex identification, and male cells can be selected early, which saves a significant amount of time at the early stage of culture.

Secondary selection can be achieved by, for example, confirmation of the number of chromosome in accordance with the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell is again cloned to a normal cell (e.g., in mouse cells having the number of chromosomes being 2n = 40) after the gene of the ES cells is rendered knockout.

Although the embryonic stem cell line thus acquired shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and 90% air) in the presence of LIF (1-10000 U/ml) on appropriate feeder cells such as STO fibroblasts, treated with a trypsin/EDTA solution (normally about 0.001 to about 0.5% trypsin/about 0.1 to about 5 mM EDTA, preferably about 0.1% trypsin/1 mM EDTA) at the time of passage to obtain separate single cells, which are then seeded on freshly prepared feeder cells. This passage is normally conducted every 1 to 3 days; it is desirable that cells be observed at passage and cells found to be morphologically abnormal in culture, if any, be abandoned.

By allowing ES cells to reach a high density in mono-layers or to form cell aggregates in suspension under appropriate conditions, it is possible to spontaneously differentiate them to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expressing the DNA of the present invention, which are obtainable from the differentiated ES cells of the present invention, are useful for studying the functions of the protein of the present invention cytologically or molecular biologically.

The non-human mammal deficient in expressing the DNA of the present invention can be identified from a normal animal by measuring the amount of mRNA in the subject animal by a publicly known method, and indirectly comparing the degrees of expression.

As the non-human mammal, the same examples *supra* apply.

With respect to the non-human mammal deficient in expressing the DNA of the present invention, the DNA of the present invention can be made knockout by transfecting a targeting vector, prepared as described above, to mouse embryonic stem cells or oocytes thereof, and conducting homologous recombination in which a targeting vector DNA sequence, wherein the DNA of the present invention is inactivated by the transfection, is replaced with the DNA of the present invention on a chromosome of mouse embryonic stem cell or its embryo.

The knockout cells with the DNA of the present invention can be identified by the Southern hybridization analysis with a DNA fragment on or near the DNA of the present invention as a probe, or by the PCR analysis using as primers a DNA sequence on the targeting vector and another DNA sequence which is not included in the targeting vector. When non-human mammalian embryonic stem cells are used, a cell line wherein the DNA of the present invention is inactivated by homologous recombination is cloned; the resulting cloned cell line is injected to, e.g., a non-human mammalian embryo or blastocyte, at an appropriate stage such as the 8-cell stage. The resulting chimeric embryos are transplanted to the uterus of the pseudopregnant non-human mammal. The resulting animal is a chimeric animal composed of both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring acquired by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between the heterozygotes.

When an egg cell is used, a DNA solution may be injected, e.g., to the nucleus of the egg cell by microinjection thereby to obtain a transgenic non-human mammal having a targeting vector introduced in a chromosome thereof. From such transgenic non-human mammals, those having a mutation at the locus of the DNA of the present invention can be obtained by selection based on homologous recombination.

As described above, individuals in which the DNA of the present invention is rendered knockout permit passage rearing under ordinary rearing conditions, after the individuals obtained by their crossing have proven to have been knockout.

Furthermore, the genital system may be obtained and maintained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal deficient in expression of the DNA of the present invention lacks various biological activities that can be derived from the protein of the present invention, such an animal can be a disease model suspected of inactivated biological activities of the protein of the present invention and thus, offers an effective study to investigate causes for and therapy for these diseases.

### [7a] Method for screening of compounds having therapeutic/prophylactic effects for diseases caused by deficiency, damages, etc. of the DNA of the present invention

The non-human mammal deficient in expressing the DNA of the present invention can be employed for the screening of compounds having therapeutic/prophylactic effects for diseases (e.g., diseases characterized by cardiac hypofunction (cardiac diseases) such as post myocardial infarction heart failure or angina pectoris, cardiomyopathy, and heart failure induced by diseases such as angina pectoris, cardiomyopathy, etc.), which are caused by deficiency, damages, etc. of the DNA of the present invention.

That is, the present invention provides a method of screening a compound or its salt having therapeutic/prophylactic effects for diseases caused by deficiency, damages, etc. of the DNA of the present invention, which comprises administering a test compound to the non-human mammal deficient in expressing the DNA of the present invention and observing/measuring a change occurred in the animal.

As the non-human mammal deficient in expressing the DNA of the present invention, which can be employed for the screening method, the same examples as given hereinabove apply.

Examples of test compounds include peptides, proteins, non-peptide compounds, synthetic compounds, fermentation products, cell extracts, plant extracts, animal tissue extracts, blood plasma and the like and these compounds may be novel compounds or publicly known compounds.

Specifically, the non-human mammal deficient in expressing the DNA of the present invention is treated with a test compound, comparison is made with an intact animal for control and a change in each organ, tissue, disease conditions, etc. of the animal is used as an indicator to assess the therapeutic/prophylactic effects of the test compound.

For treating an animal under test with a test compound, for example, oral administration, intravenous injection, etc. are applied and the treatment is appropriately selected depending upon conditions of test animal, properties of test compound, etc. Furthermore, an amount of test compound administered can be selected depending on administration route, nature of test compound, and the like.

For example, in the case of screening a compound having a therapeutic/prophylactic effect for, e.g., heart failure, the non-human mammal deficient in expressing the DNA of the present invention is subjected to a pressure overloading treatment, e.g., by heart failure-forming operation such as coronary stenosis operation, etc., animal receives a test compound before or after the pressure overloading treatment and, cardiac hemodynamics, body weight change, etc. of the animal is measured with passage of time.

Also, cardiac hemodynamics are assessed or the presence or absence of cardiac hypertrophy is examined in a pressure-overloaded model with cardiac hypertrophy induced by constriction of the abdominal aorta, etc.

In the screening method *supra,* when a test compound is given to an animal under test and found to reduce cardiac hypertrophy of the animal to at least about 10%, preferably at least about 30% and more preferably at least about 50%, the test compound can be selected as a compound having a therapeutic/prophylactic effect for heart failure.

The compound obtained using the above screening method is a compound selected from the test compounds described above and exerts a therapeutic/prophylactic effect for the diseases (e.g., heart failure, etc.) caused by deficiencies, damages, etc. of the protein of the present invention. Therefore, the compound can be employed as a safe and low toxic drug for the treatment and prevention of these diseases. Furthermore, compounds derived from such a compound obtained by the screening *supra* can be likewise employed.

The compound obtained by the screening method above may be used in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

A pharmaceutical containing the compound obtained by the above screening methods or salts thereof may be manufactured in a manner similar to the method for preparing the composition comprising the protein of the present invention described hereinabove. Since the pharmaceutical composition thus obtained is safe and low toxic, it can be administered to mammals (e.g., human, rat, mouse, guinea pig, rabbit, sheep, swine, bovine, horse, cat, dog, monkey, etc.).

Although the amount of the compound or its salt to be administered varies depending upon target disease, subject to be administered, route of administration, etc., when the compound is orally administered for the treatment of heart failure, the compound is generally administered to an adult (as 60 kg body weight) in a dose of about 0.1 to about 100 mg, preferably about 1.0 to about 50 mg, more preferably about 1.0 to about 20 mg per day. For parenteral administration, a single dose of the compound varies depending upon subject to be administered, target disease, etc., but when the compound is administered to an adult (as 60 kg body weight) in the form of an injectable preparation for the purpose of treating heart failure, it is generally advantageous to administer the compound intravenously in a dose of about 0.01 to about 30 mg, preferably about 0.1 to about 20 mg, more preferably about 0.1 to about 10 mg per day. As for other animals, the composition can be administered in the above amount with converting it into that for the body weight of 60 kg.

### [7b] Method of screening a compound that promotes the activity of a promoter to the DNA of the present invention

The present invention provides a method of screening a compound or its salt that promotes the activity of a promoter to the DNA of the present invention, which comprises administering a test compound to a non-human mammal deficient in expressing the DNA of the present invention and detecting expression of the reporter gene.

In the screening method described above, the non-human mammal deficient in expressing the DNA of the present invention is selected from the aforesaid non-human mammal deficient in expressing the DNA of the present invention, as an animal, in which the DNA of the present invention is inactivated by introducing a reporter gene and the reporter gene can be expressed under control of a promoter to the DNA of the present invention.

The same examples of the test compound apply to specific compounds used for the screening.

As the reporter gene, the same specific examples described above apply, and β-galactosidase gene (lacZ), soluble alkaline phosphatase gene, luciferase gene and the like are preferably employed.

Since a reporter gene is present under control of a promoter to the DNA of the present invention in the non-human mammal deficient in expressing the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from *Escherichia coli,* β-galactosidase is expressed in a tissue where the protein of the present invention should originally be expressed, instead of the protein of the present invention. Thus, the state of expression of the protein of the present invention can be readily observed *in vivo* of an animal by staining with a reagent, e.g., 5-bromo-4-chloro-3-indolyl-β-galactopyranoside (X-gal), which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method *supra* are compounds that are selected from the test compounds described above and that promote or inhibit the promoter activity to the DNA of the present invention.

The compound obtained by the screening method above may be used in the form of salts with physiologically acceptable acids (e.g., inorganic acids) or bases (e.g., organic acids), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Since the compounds or salts thereof that promote the promoter activity to the DNA of the present invention can promote the expression of the protein of the present invention, or can promote the function of the protein, they are useful as a drug such as a safe and low toxic therapeutic/preventive agent for, e.g., diseases characterized by cardiac hypofunetion (heart diseases) such as post myocardial infarction heart failure or angina pectoris, cardiomyopathy, and heart failure induced by diseases such as angina pectoris, cardiomyopathy, etc.

A pharmaceutical comprising the compounds or salts thereof obtained by the screening method *supra* may be manufactured in a manner similar to the method for preparing the pharmaceuticals containing the protein or its salt of the present invention described above.

Since the pharmaceutical preparation thus obtained is safe and low toxic, it can be administered to mammal (e.g., human, rat, mouse, guinea pig, rabbit, sheep, swine, bovine, horse, cat, dog, monkey, etc.).

The dose of the compound or salts thereof varies depending on target disease, subject to be administered, route for administration, etc.; for example, when the compound that promotes the promoter activity to the DNA of the present invention is orally administered for the purpose of treating heart failure, the dose is normally about 0.1 to about 100 mg, preferably about 1.0 to about 50 mg, more preferably about 1.0 to about 20 mg per day for adult (as 60 kg body weight). In parenteral administration, a single dose of the compound varies depending on subject to be administered, target disease, etc. but when the compound that promotes the promoter activity to the DNA of the present invention is administered in the form of injectable preparation for the purpose of treating heart failure, it is advantageous to administer the compound intravenously to adult (as 60 kg body weight) at a single dose of about 0.01 to about 30 mg, preferably about 0.1 to about 20 mg, more preferably about 0.1 to about 10 mg per day. For other animal species, the corresponding dose as converted per 60 kg weight can be administered.

As stated above, the non-human mammal deficient in expressing the DNA of the present invention is extremely useful for screening of the compound or its salt that promotes the activity of a promoter to the DNA of the present invention and can greatly contribute to the elucidation of causes for various diseases suspected of deficiency in expressing the DNA of the present invention and for the development of prophylactic/therapeutic agent for these diseases.

Furthermore, a so-called transgenic animal (gene transfected animal) can be prepared by using a DNA containing a promoter region of the protein of the present invention, ligating genes encoding various proteins downstream and injecting the same into egg cell of an animal. It is then possible to synthesize the protein specifically and study its activity *in vivo.* When an appropriate reporter gene is ligated to the promoter site above and a cell line that expresses the gene is established, the resulting system can be utilized as the survey system for a low molecular compound having the action of specifically promoting or inhibiting the *in vivo* productivity of the protein *per se* of the present invention. By analyzing the promoter part, it is also possible to find a new cis-element or a transcription factor capable of binding thereto.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.
- DNA: : deoxyribonucleic acid
- CDNA: : complementary deoxyribonucleic acid
- A: : adenine
- T: : thymine
- G: : guanine
- C: : cytosine
- RNA: : ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP: : adenosine triphosphate
- EDTA: : ethylenediaminetetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid

Also, substituents, protecting groups, and reagents frequently used in this specification are presented as the codes below.
- Me: : methyl group
- Et: : ethyl group
- Bu: : butyl group
- Ph: : phenyl group
- TC: : thiazolidine-4(R)-carboxamide group
- Tos: : p-toluenesulfonyl
- CHO: : formyl
- Bzl: : benzyl
- Cl₂Bzl: : 2,6-dichlorobenzyl
- Bom: : benzyloxymethyl
- Z: : benzyloxycarbonyl
- Cl-Z: : 2-chlorobenzyl oxycarbonyl
- Br-Z: : 2-bromobenzyl oxycarbonyl
- Boc: : t-butoxycarbonyl
- DNP: : dinitrophenyl
- Trt: : trityl
- Bum: : t-butoxymethyl
- Fmoc: : N-9-fluorenyl methoxycarbonyl
- HOBt: : 1-hydroxybenztriazole
- HOOBt: : 3,4-dihydro-3-hydroxy-4-oxo-1,2,3-benzotriazine
- HONB: : 1-hydroxy-5-norbornene-2,3-dicarboxyimide
- DCC: : N,N'-dicyclohexylcarbodiimide

The sequence identification numbers in the sequence listing of the specification indicates the following sequence, respectively.

### [SEQ ID NO:1]

This shows the amino acid sequence of rat CARP.

### [SEQ ID NO:2]

This shows the base sequence of DNA encoding rat CARP having the amino acid sequence represented by SEQ ID NO: 1.

### [SEQ ID NO:3]

This shows the base sequence of a primer used in EXAMPLE 3.

### [SEQ ID NO:4]

This shows the base sequence of a primer used in EXAMPLE 3.

### [SEQ ID NO:5]

This shows the base sequence of a primer used in EXAMPLE 4.

### [SEQ ID NO:6]

This shows the base sequence of a primer used in EXAMPLE 4.

### [SEQ ID NO:7]

This shows the base sequence of a primer used in EXAMPLE 5.

### [SEQ ID NO:8]

This shows the base sequence of a primer used in EXAMPLE 5.

### [SEQ ID NO:9]

This shows the base sequence of a primer used in EXAMPLE 7.

### [SEQ ID NO:10]

This shows the base sequence of a primer used in EXAMPLE 7.

### [SEQ ID NO: 11]

This shows the base sequence of a primer used in EXAMPLE 7.

### [SEQ ID NO:12]

This shows the base sequence of a primer used in EXAMPLE 7.

### [SEQ ID NO:13]

This shows the base sequence of a primer used in EXAMPLE 7.

### [SEQ ID NO:14]

This shows the base sequence of a primer used in EXAMPLE 7.

### [SEQ ID NO:15]

This shows the base sequence of a primer used in EXAMPLE 7.

### [SEQ ID NO:16]

This shows the base sequence of a primer used in EXAMPLE 7.

### [SEQ ID NO:17]

This shows the base sequence of a primer used in EXAMPLE 7.

### [SEQ ID NO:18]

This shows the base sequence of a primer used in EXAMPLE 7.

### [SEQ IDNO:19]

This shows the base sequence of a primer used in EXAMPLE 7.

### [SEQ ID NO:20]

This shows the base sequence of a primer used in EXAMPLE 7.

### EXAMPLES

The present invention is described below more specifically, with reference to EXAMPLES, but is not deemed to be limited thereto. The gene manipulation procedures using *Escherichia coli* were carried out according to the methods described in Molecular Cloning, 2nd., J. Sambrook et al., Cold Spring Harbor Lab. Press, 1989.

### EXAMPLE 1

### Preparation of model rat with myocardial infarction

Wistar male rats (11 weeks old: weighing 300-400 g) were anesthetized with pentobarbital (50 mg/kg, i. p.) in accordance with the report by Watanabe et al. (Circulation Research, 69, 370-377, 1991), and a median thoracotomy was performed under artificial respiration. After pericardiectomy, the heart was exposed to allow visualization. A suture needle (Elp Co., 5-0 silk) was looped around the left anterior descending branch of the coronary artery at its origin, and the coronary artery was tied by the silk suture together with the myocardium. The chest was then closed. In the sham-operated group, the chest was closed without ligation. After recovery from anesthesia, the animals were placed in normal feeding.

### EXAMPLE 2

### Extraction of total RNA

At postoperative 1, 8, 20 and 30 weeks, rats were anesthetized with pentobarbital for thoracotomy, and the heart was removed. Then, the coronary artery was subjected to retrograde perfusion from the aorta with physiological saline to wash blood away. Tissues other than the left ventricle were withdrawn from the heart removed with scissors, and formation of infarcts was confirmed. Then the infarct area (scar-formed site) was removed to leave non-infarct area alone. The non-infarct area was minced with scissors followed by extraction of total RNA using ISOGEN (manufactured by Wako Pure Chemical Industries Co., Ltd.).

### EXAMPLE 3

### Cloning of rat CARP gene

In order to remove genomic DNA from the total RNA, DNA degradation was performed using enzyme set for DD (manufactured by Takara Shuzo Co., Ltd.). Then, differential display (DD) was carried out using Fluorescenece Differential Display Kit Fluorescein version (manufactured by Takara Shuzo Co., Ltd.). The target tissue used was the total RNA derived from the left ventricle at postoperative 8 weeks in the sham operation group. As the result, a band showing a marked increase in the tissues at postoperative 1, 20 and 30 but conversely showing a decrease at postoperative 8 weeks was noted, when compared to the control tissue. This band was cut out of the acrylamide gel with a cutter, and suspended in a sterile distilled water. The suspension was heated at 95°C for 10 minutes to extract a gene fragment from the gel. Next, after re-amplification by PCR, its DNA base sequence was decoded. Based on the thus revealed base sequence, homology survey was conducted by BlastN using Geneble Database, which is a public database. This gene fragment was identified to be the base sequence encoding rat CARP publicly known (J. B. C., 272, 22800-22808, 1997; Development, 124, 793-804, 1997). Next, cDNA library was prepared from the total RNA derived from the left ventricle at postoperative 1 week, using the reverse transcription kit of PE Applied Biosystems, and using this library as a template, PCR was carried out using 2 primers of the 5' non-translational region (SEQ ID NO:3) and 3' non-translational region (SEQ ID NO:4) of the CARP gene. Thus, the full-length rat CARP gene was acquired (SEQ ID NO:1). The reaction was carried out on the Thermal Cycler gene amp PCR system 9700 (manufactured by Perkin-Elmer, Inc.) using Pfu DNA polymerase (manufactured by Toyobo Co., Ltd.), by repeating 33 cycles of one cycle set to include 95°C for 10 seconds, 66°C for 30 seconds and 72°C for 3 minutes. The thus acquired full-length CARP gene was cloned to pCR II-Blunt TOPO vector using Zero Blunt TOPO PCR Cloning Kit (manufactured by Invitrogen, Inc.). Using synthetic primers publicly known (T7 primer and SP6 primer), the cloned product was further applied to Cycle Sequencing Kit of PE Applied Biosystems and analyzed by a fluorescent DNA sequencer (ABI PRISM 377, manufactured by Perkin-Elmer, Inc.). The gene fragment was identified to be rat CARP gene.

### EXAMPLE 4

### Analysis of tissue distribution of CARP gene in normal rat

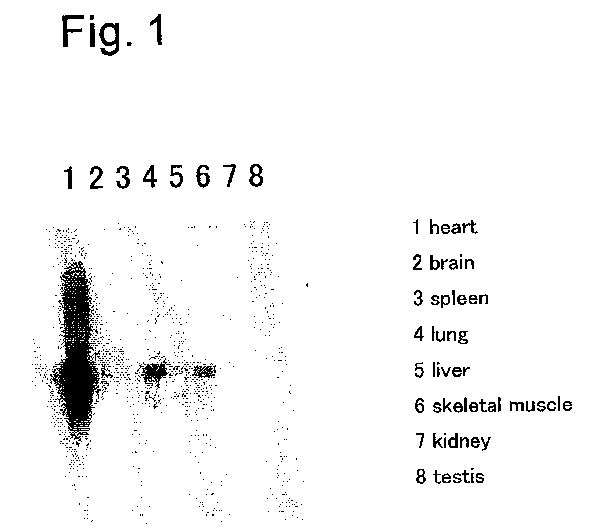

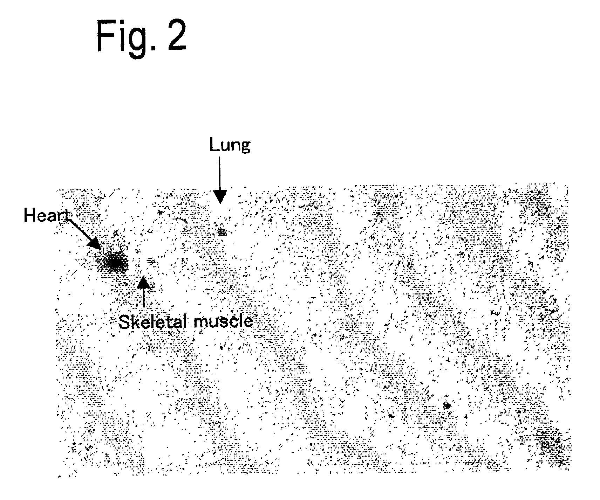

In order to obtain a probe for northern blotting, PCR was carried out by the procedures of EXAMPLE 3, using as a template the CARP gene obtained in EXAMPLE 3 and using SEQ ID NOS:5 and 6. Rat MTN Blot and mouse RNA Master Blot manufactured by Clontech Laboratories, Inc. were used as membranes for the northern blotting. Using Express Hyb Hybridization solution (manufactured by Clontech Laboratories, Inc.) as a hybridization solution, hybridization was performed at 68°C. On the other hand, the CARP gene fragment prepared as a probe was labled with [α-³²P] dCTP and BcaBEST Labeling Kit (manufactured by Takara Shuzo Co., Ltd.). The hybridization was carried out in Express Hyb Hybridization solution (manufactured by Clontech Laboratories, Inc.) containing the labeled probe under conditions of 68°C for an hour. The membrane was finally washed at 50°C in 0.1 x SSC, 0.1% SDS solution. For detection, BAS-2000 (manufactured by Fuji Photo Film Co., Ltd.) was used. As the result (FIGS. 1 and 2), the heart was found to be a major expression site of the rat CARP gene, and its expression was noted also in lung and skeletal muscle, though it was weak.

### EXAMPLE 5

### Analysis of change of CARP gene with passage of time in model rat with myocardial infarction

Using TaqMan Reverse Transcription Reagents (manufactured by PE Applied Biosystems), cDNA was synthesized from the total RNA derived from the left ventricular non-infarct area of rats at the postoperative 1, 8, 20 and 30 weeks of myocardial infarction-forming surgery explained in EXAMPLE 2 and the total RNA derived from the left ventricle of sham-operated rats at the postoperative 8 weeks of myocardial infarction-forming surgery used as control. Next, a quantification of copy number of CARP gene by PCR was performed by ABI Prism 7700 Sequence Detection System using SEQ ID NOS: 5 and 7 as CARP-specific primers and using a fluorescence-labeled form of SEQ ID NO:8 as a probe (manufactured by PE Applied Biosystems). The reaction was carried out using TaqMan PCR Core Reagents Kit (manufactured by PE Applied Biosystems), and all of the procedures were carried out in accordance with the instructions attached. Standard used for quantification was prepared as follows. Using TaqMan Reverse Transcription Reagents (manufactured by PE Applied Biosystems), cDNA was synthesized from the total RNA derived from the left ventricular non-infarct area of rats on 1 week after the myocardial infarction-forming surgery. Next, PCR was carried out using SEQ ID NOS: 5 and 7 as CARP-specific primers, and the resulting gene fragment having a partial sequence of the CARP gene was made its standard. Furthermore, the number of copies calculated was corrected by the copy number of glycerol-3-phosphate dehydrogenase as an internal control computed as in the CARP gene copy number, and compared to the control tissue. The data is shown in its fold increase (FIG. 3). The results revealed that the CARP gene somewhat increased at postoperative 1 week (5.9 times), then decreased at postoperative 8 weeks (0.47 time) and markedly increased at postoperative 20 and 30 weeks (21.4 and 12.3 times, respectively).

It is considered that the 1 week period immediately after the operation would be a period during which infarcts are being formed, and assumed that cardiomyocytes in the downstream region from the constricted coronary artery would rapidly become extinct and exfoliated to cause inflammation due to infiltration of lymphocytes. It is also considered that since death was observed immediately before the postoperative 20 to 30 weeks, a compensatory mechanism would work during the postoperative 8 weeks, and during or after the postoperative 20 weeks, the compensatory mechanism would not be sufficiently activated or decompensation would take place due to excessive compensatory mechanism. Thus, the postoperative 1 week, 8 weeks and 20 weeks or thereafter were thought to be acute, chronic and end stages, respectively. The compensatory mechanism involved in the transition from myocardial infarction to heart failure is considered to be activated as follows. When cardiomyocytes are exfoliated (necrosis or apoptosis), the remaining cardiomyocytes will be hypertrophied in order to compensate for the function possessed by the lost cardiomyocytes by the entire heart, thus causing reconstruction (heart remodeling) accompanied by cardiac dilation or fibrosis. By this process, the cardiac function will be functionally compensated, but on the other hand, it is considered that this cardiac remodeling or excessive compensatory mechanism itself would be at risk of developing heart failure (NAIKA, 79, 2-20, 1997). However, any molecule involved in decompensation *per se* has not yet been identified, and thus its mechanism is unknown, either. As shown in EXAMPLE described above, genes were cyclopaedically studied in terms of expression levels during the process from the chronic to end stages using the aforesaid rat model with myocardial infarction and as a result, the CARP gene was discovered as a gene with a marked change in its expression level. The gene increased at the chronic stage and decreased prominently at the end stage. Reportedly, this gene suppresses the expression of atrial natriuretic peptide, which is thought to act protectively on the heart, and troponin C, which is one of contractile proteins. Moreover, the gene is considered to be a transcription factor that plays an important role in cardiac development. While a targeting gene of the CARP gene product has not sufficiently been clarified yet, it is speculated that it will be a cardiac-specific gene required for maintenance of cardiac function (J. B. C., 272, 22800-22808, 1997; Development, 124, 793-804, 1997; Development, 126, 4223-4234, 1999). Recently, the results obtained by cyclopaedical analysis of gene expression in a model rat with myocardial infarction were reported (Circulation Research, 86, 939, 2000) and indicate a weak augmentation in expression of the CARP gene at the acute stage, as discovered in the present invention. However, these results are based on observation up to postoperative 16 weeks, and are silent on a marked change in expression between the chronic and end stages. Besides, the gene expression analysis applied to the journal above is the differential hybridization method using gene chips. It would appear that this method is obviously less sensitive than the TaqMan PCR method shown in EXAMPLE above. For this reason, it was assumed that reduction in expression of the CARP gene at the chronic stage could not be detected.

A detailed expression profile of the CARP gene clarified in EXAMPLE described above is considered to be as follows. That is, the decreased expression at the chronic stage results in release of the suppression system by the CARP gene product. As a result, the increased expression of cardiac-specific gene group is considered to take place, that is, it is believed that a compensatory mechanism would be reinforced. When the stage approaches the end, its expression increases to the contrary and as a result, it is considered that the compensatory mechanism would be deteriorated, leading to decompensation; thus the pumping function would be deteriorated to death. It is critical for prophylactic/therapeutic agents for cardiac diseases to prevent both an excessive compensatory mechanism and decompensation. It is important in solving this problem to appropriately control the expression of CARP gene product. It appears that the decreased expression would induce an excessive compensatory mechanism and the increased expression would accelerate decompensation. Thus, drugs for regulating the CARP gene expression or the gene product function are useful as novel prophylactic/therapeutic agents for cardiac diseases.

### REFERENCE EXAMPLE 1

### Preparation of primary neonatal rat cardiomyocyte

Throughout the experiment, neonatal Wistar rats (within 1 day after birth) prepared by Charles River Japan, Inc. were used. Neonatal rats under ether anesthesia were sterilized with 70% ethanol followed by withdrawal of the heart with tweezers. The heart withdrawn was washed with phosphate buffered saline (manufactured by Takara Shuzo Co., Ltd., T900) and cut into slices with surgical scissors.

The slices were washed 4 or 5 times with phosphate buffered saline to remove most of non-cardiomyocytes derived from blood, which was then digested with enzyme. To wash and remove the blood-derived non-cardiomyocytes was carried out by putting the heart tissue slices in a cell strainer (manufactured by Falcon Co., Ltd.) and washing them in phosphate buffered saline. The digestion with enzyme was carried out by adding 5 ml of enzyme solution (a solution of 1.25 mg of trypsin (manufactured by Difco) and 0.25 mg of collagenase (manufactured by Sigma) in 1 ml of PBS (-)) to the tissue slices corresponding to 10 neonatal rats, treating for 15 minutes, supplementing 2.5 ml of the enzyme solution twice every 15 minutes and agitating with a stirrer while keeping at 37°C. In this process, individual cell was separated from the tissue slices. After completion of the reaction, Medium 199 (manufactured by Difco) containing 10% fatal calf serum (manufactured by Bio wicker) was added in a half volume of the enzyme solution to terminate the enzyme reaction. After filtering through the cell strainer, the filtrate was centrifuged at 400 x g for 5 minutes to collect cells. Next, the cells corresponding to 10 neonatal rats were suspended in 50 ml of Medium 199 containing 10% fatal calf serum, and 10 ml each of the suspension was inoculated on a 100 ml Petri dish (manufactured by Iwaki Glass Co., Ltd.) followed by incubation for an hour in a CO₂ incubator set at 5% CO₂ and 37°C. The suspended cells were recovered and filtered through the cell strainer. The filtrate was centrifuged at 400 x g for 5 minutes to collect cardiomyocytes.

Next, cardiomyocytes corresponding to 10 neonatal rats were suspended in 2 ml of a low isotonic solution (obtained by dissolving 8.29 g of NU₄Cl, 1.0 g of KHCO₃ and 37 mg EDTA/2Na in 1 liter of water). The suspension was allowed to stand for 3 minutes to homogenate erythrocytes. After 10 ml of Medium 199 containing 10% fatal calf serum was added to the homogenate, the mixture was centrifuged at 400 x g for 5 minutes to collect cardiomyocytes. The cardiomyocytes were suspended in Medium 199 containing 10% fatal calf serum followed by filtration through the cell strainer.

An aliquot of the cell suspension obtained was taken up and 0.3% trypan blue was added thereto. After gently mixing them, the count of cardiomyocytes was determined using a haemocytometer. The cardiomyocytes almost free of erythrocytes were obtained in 1.4 x 10⁷ from the heart corresponding to 10 neonatal rat.

### EXAMPLE 6

### Formation of expression profile

The primary cardiomyocytes prepared as described above were added to a 12-well plate in 300,000 cells/well and cultured for 18 hours in a 10% serum-supplemented medium. Then, the experiment was carried out in the same medium after removing the serum. In the measurement, the expression level was quantified by the TaqMan quantification method described in EXAMPLE 5. By removing the serum, apoptosis could be induced, but an increased expression of the CARP gene by about twice was observed 24 hours later, when compared to 4 hours after the incubation. In addition, norepinephrine or endothelin, which was known to be a humoral factor to increase its secretion in heart failure, was added to 10 nM, 100 nM or 1000 nM medium, and an increase of expression by about 3 times at maximum was noted with both factors. Since these humoral factors have an activity of promoting hypertrophy of cardiomyocytes, it was revealed that the CARP gene expression increases in response to hypertrophic activation by these factors. According to this system, the CARP gene expression can be quantitatively determined in various hypertrophic stimuli or at an apoptosis-inducing stage, and can be used for screening of a CARP gene expression regulator.

### EXAMPLE 7

### Preparation of recombinant adenovirus

Cloning of the rat CARP cDNA cloned in EXAMPLE 3 to a virus vector was carried out by the adeno-custom service provided by Takara Shuzo Co., Ltd. Using the adenovirus expression vector kit (6150) manufactured by Takara Shuzo Co., Ltd., cloning was carried out in accordance with the protocol attached to produce CARP sense strand expression adenovirus. Preparation of virus purified to high purity and titer assay were carried out by publicly known methods (JIKKEN IGAKU, extra issue, SHIN-IDENSHI KOGAKU HANDBOOK, pages 238-244) to finally prepare a purified virus stock solution in a concentration of 3.6 x 10¹⁰ pfu/ml. Null virus (virus containing no insert) was prepared as described above, and a purified virus stock solution was finally prepared in a concentration of 1.3 x 10⁹ pfu/ml.

### Surgical preparation

Following the report by Watanabe et al. (Circulation Research, 69, 370-377, 1991), Wistar male rats (8 weeks old, weighing 250-300 g) were anesthetized with sodium pentobarbital (50 mg/kg, i. p.). Body fur at the abdominal side of the chest and cervical regions was clipped with a hair clipper and laid down dorsally on an operating table. Kite strings were crossbridged between the four limbs and maxillary incisors to secure the animal on the operating table and sterilize the incised zone with 80% ethanol solution. The skin at the cervical region and the muscle therebeneath were dissected to expose the trachea. Under visual observation, a 14G stent case (Terumo Corporation) was orally intubated into the trachea and connected to an artificial respirator. Lips and the stent case were connected through a suture silk (Natsume, 3-0 braided silk suture) so that the artificial respirator was not dislocated.

### Thoracotomy and exposure of the heart

A median thoracotomy was performed at the skin and trunk dermal muscle by about 5 mm left from the rat sternum to achieve incision of about 4 cm. Next, the area around the sternum from the second to fifth costicartilage located just below the incised area was incised. The intercostal muscles were detached with surgical scissors. After hemostasis, the sternum was opened by about 3 cm using a thoraco-opener. Furthermore, the pericardium was bluntly detached with a cotton swab and opened to visualize the heart.

### Placement of silk suture for thoracic aorta stenosis

The thymus gland was uplifted to detach the connective tissue of thoracic aorta around the heart with a cotton swab, extending out of the heart. A surgical suture (Natsume, 5-0 braided silk suture) was picked up with tip-curved aniridia tweezers and looped around the back from the left to right thoracic aorta to place the silk suture for stenosis in the thoracic aorta. The silk suture thus arranged was passed through a Teflon tube for stenosis with an inner diameter of about 1 mm and was inserted to tighten with the Teflon tube so that the thoracic aorta could be occluded.

### Administration of viral solution into the left ventricle

Using a cotton swab, the heart was reversed around the auricle from the apex to move the heart toward the abdominal side. An aqueous solution of the CARP gene expression viral vector having a high purity was diluted with physiological saline to prepare a 10⁸ pfu/ml solution. The solution was weighed into a 1 ml syringe (Terumo Corporation) tipped with a 27G needle. Virus was administered in 2 x 10⁸ pfu/kg body weight. The procedure was as follows. The syringe was inserted into the left ventricle while avoiding the coronary artery/vein, and then the Teflon tube was tightened to produce thoracic aorta stenosis. After confirmation of the stenosis, the viral solution was gradually administered into the left ventricle to perfuse into the left ventricle. Since the left ventricle was expanded while administration, the Teflon tube was loosened once in every about 5 seconds for global perfusion of the viral solution to prevent any secondary influence that might be caused by the dilated left ventricle. The operation of this administration and loosening of the tightened Teflon tube was repeated 3 times in total to achieve perfusion of the whole volume of viral solution into the left ventricle.

### Withdrawal of suture silk for thoracic aorta stenosis and closure of the chest

One loop of the silk suture for thoracic aorta stenosis was cut off and another loop was pulled out to withdraw the silk suture. After blood in the thoracic cavity was absorbed into a cotton swab and disposed, the chest wall and skin were stitched with the suture silk in an about 5 mm interval. The skin and musculature incisions made in the cervical intubation were also closed by the same suture. After labored respiration by the artificial respirator for an hour or longer immediately after the surgery, spontaneous breathing of the animal was confirmed in the state that the animal was emerged from anesthesia, and the tube was pulled out. The postoperative animal was encaged in a breeding cage.

### Measurement of cardiac function

Following the report by Watanabe et al. (Circulation Research, 69, 370-377, 1991), the cardiac function was assessed as follows. The rat at postoperative 1 week was weighed and anesthetized with sodium pentobarbital (50 mg/kg, i. p.). Body fur at the abdominal side of the chest and cervical regions was clipped with a hair clipper and laid down dorsally on an operating table. The cervical region at the abdominal side was incised with surgical scissors and the muscle therebeneath was incised with tweezers to detach from the connective tissue the cervical aorta located transversely in a plane perpendicular to the trachea, A silk suture (Natsume, 3-0 braided silk suture) was looped around the blood vessel to achieve a ligature at the head side and hemostasis at the abdominal side with arterial forceps. The blood vessel was then incised with surgical scissors, Miller catheter (manufactured by Miller Inc., 2F, 140 cm, Catalog No. 800-0509) was inserted into the vessel and a tip was furnished to the left venticle. The Miller catheter was connected to a polygraph manufactured by NEC Corporation. After the Miller catheter was securely inserted into the left ventricle by monitoring a pressure change on a CRT (Nihon Koden, CRT monitor). the intraventricular pressure was measured. A mean blood pressure was determined at the point when inserted into the blood vessel. The measurement data of intraventricular pressure, heart beats and intraventricular pressure change rate (LV+dp/dt and LV-dp/dt) were calculated and computed by Mac Labs. The Miller catheter was calibrated at 2 points of 0 mmHg and 100 mmHg immediately before the onset of experiment, using a caliber (manufactured by Omeda Co., Xcaliber, PN072490-000-000). As the result, the intraventricular pressure change rate (LV+dp/dt) significantly decreased in the CARP expression virus transfection group, when compared to the null virus administration group. No significant change was noted in other parameters.

### Measurement of left ventricular weight

After completion of the cardiac function assessment, thoracotomy was performed. The heart was picked up with tweezers to remove blood vessels and connective tissues, and the heart was isolated. The heart was washed in physiological saline. A syringe was inserted into the aorta attached to the isolated heart, and physiological saline was perfused throughout the heart to remove blood. Then, the atrial was removed to weigh only the ventricular tissue. The heart weight was determined by correcting with body weight. As the result, no significant difference was observed between the CARP expression virus administration group and the null virus administration group.

The heart after weighing was vertically sliced with surgical scissors into the left and right ventricles equally divided. Thus, samples were prepared for tissue analysis and for gene analysis. The individuals postoperatively survived were 11 rats in the null virus administration group and 7 rats in the CARP expression virus administration group. However, 2 rats in the null virus administration group were died during the cardiac function measurement, and the gene analysis and the heart weight measurement were made with 11 rats in the null virus administration group, but the cardiac function measurement was made with 9 rats from the null virus administration group.

### Analysis of gene expression

### Preparation of total RNA

The right ventricular segment was removed from the sample for gene analysis, and the left ventricle alone was minced with surgical scissors. Then, total RNA was extracted with ISOGEN (manufactured by Wako Pure Chemical Industries, Ltd.).

### Synthesis of cDNA

Using TaqMan Reverse Transcription Reagents (manufactured by PE Applied Biosytems), cDNA was synthesized from the total RNA.

### Expression analysis by TaqMan PCR

For expression analysis, the copy number of CARP gene was examined for the purpose of ascertaining the CARP gene transfection. In addition, the copy number of ANP as a marker gene for cardiac hypertrophy, myosin light chain, myosin heavy chain and cardiac action as contractile proteins was examined.

The copy number of CARP gene by PCR was quantified in ABI Prism 7700 Sequence Detection System using SEQ ID NOS: 5 and 7 as CARP-specific primers described in EXAMPLE 2 and using a fluorescence-labeled form of SEQ ID NO: 8 (manufactured by PE Applied Biosystems) as a probe. The reaction was carried out all in accordance with the procedures as instructed in the protocol attached, using TaqMan PCR Core Reagents Kit (manufactured by PE Applied Biosystems). Standard for quantification was prepared by the method described below. Using TaqMan Reverse Transcription Reagents (manufactured by PE Applied Biosystems), cDNA was prepared from the total RNA derived from the non-infarcted area of left ventricle in myocardial infarction-formed postoperative 1 week rat. Next, PCR was carried out using SEQ ID NO:5 and SEQ ID NO:7 as CARP-specific primers, and the resulting gene fragment having a partial sequence of the CARP gene was made its standard. Furthermore, the copy number computed was corrected by the copy number of glycerol-3-phosphate dehydrogenase as an internal control computed as in the CARP gene copy number, and compared to the null virus administration group. The results revealed that the CARP expression virus administration group expressed the CARP gene in a significantly high level.

ANP was analyzed using SEQ ID NO:9 and SEQ ID NO: 10 as specific primers and SEQ ID NO: 17 (manufactured by PE Applied Biosystems) as a probe. Myosin light chain, myosin heavy chain and cardiac actin were analyzed, respectively, using SEQ ID NO:12 and SEQ ID NO:13 as specific primers and SEQ ID NO:14 (manufactured by PE Applied Biosystems) as a probe, using SEQ ID NO:15 and SEQ ID NO:16 as specific primers and SEQ ID NO:17 (manufactured by PE Applied Biosystems) as a probe, and using SEQ ID NO: 18 and SEQ ID NO:19 as specific primers and SEQ ID NO:20 (manufactured by PE Applied Biosystems) as a probe.

As the result, any significant difference in ANP expression level was not noted between the two groups, indicating conformity to the results that there was no change in heart weight and no hypertrophy was caused. Moreover, since myosin heavy chain in the contractile proteins was significantly increased in the CARP expression virus administration group, it can be speculated that abnormality in sarcomeric organization of the myocardium would have occurred in this group.

Statistical analysis of the foregoing results was made between the null virus administration group and the CARP expression virus administration group, using a t-test. Statistical significance was taken to be P < 0.05,

### Pathological analysis

### Preparation of microscopic section

It was predicted that direct transfection of the CARP gene to the cardiomyocyte via a virus vector would cause morphological change of the cardiomyocyte. Thus, the morphological change between the CARP gene transfection group and the null transfection group was pathologically compared and studied to examine the effect of CARP gene transfection on the cardiomyocyte.

The gene was transfected by a virus vector. After the cardiac function of animal was assessed with passage of time, the entire heart was isolated and perfusion-washed with 10 ml of physiological saline using a 10 ml syringe. Then, the heart that was made a sample for tissue analysis was fixed with 10 ml of 4% paraformaldehyde phosphate buffer. The heart was further subjected to post-fixation overnight at 4°C in 4% paraformaldehyde phosphate buffer. After fixation, the tissue section was washed with distilled water and cross-sectioned into two equal sections to the long axis. The hearts were immersed continuously in 70% ethanol, in 80% ethanol, in 90% ethanol, twice in 100% ethanol and twice in xylene, all for 2 hours, to dehydrate and clear the tissue. Next, the hearts were immersed twice in about 56 paraffin for 2 hours, respectively, to prepare a paraffin block on the tissue cassette as its bed. The paraffin block was rapidly chilled in a refrigerator overnight or longer to completely solidify. The sections were, after thawing the paraffin block to room temperature, sliced in a 5 µm-thick section with a microtome. The sections were mounted while warming on a mass-coated slide glass. After drying the mounted sections on a paraffin stretcher, the sections were allowed to stand overnight in a dryer kept warm at 37°C. For further stretching, heating was continued at 60°C for an hour. Deparaffinization and general staining (hematoxylin and eosin double staining method) were carried out by routine procedures with reference to the following method (Yutaka Sano, SOSHIKIGAKU KENKYUHO, published by Nanzando, 1985). The sections after staining were dehydrated sequentially in 70% ethanol, in 80% ethanol, in 90% ethanol, twice in 100% ethanol and twice in xylene. To clear and embed was carried out with reference to the following (Yutaka Sano, SOSHIKIGAKU KENKYUHO, published by Nanzando, 1985).

### Observation of striated structure

Using an optical microscope equipped with a differential interference apparatus, the stained sections were microscopically observed in an enlarged 1000-fold magnification. A typical example of the myocardial tissue observed microscopically is described below. The cardiomyocyte of the null transfection group was abundant in eosinophilic cytoplasm, in which striated structure of the cardiomyocyte was clearly observed. On the other hand, the hearts in the CARP gene transfection group had similar orientation of cardiomyocyte, nuclear positioning and thickness of the heart wall, to the null transfection group, but the site deleted of striated structure in the cardiomyocyte was observed. Based on the foregoing, one of the causes for decreased cardiac contractility due to transfection of the CARP gene is assumed to be responsible for the deletion of striated structure.

Based on the results above, it is considered that overexpression of the CARP gene in the heart would affect to change the expression of contractile proteins leading to degeneration of the sarcomeric organization as its constitutive unit, which would cause destruction of the striated structure. For this reason, deterioration of cardiac function (cardiac contractility) appears to take place. Based on the publicly known information of CARP and its expression profile in heart failure models, it was predicted that the function would be attributable to a regulatory factor negative to the heart-specific gene and thus, its upregulated expression was assumed to cause cardiac dysfunction. However, any gene with downregulated expression *in vivo* by the CARP transfection could not be found in the heart-specific genes examined in this EXAMPLE. To the contrary, increased expression of myosin heavy chain was noted. Based on this result, the function of CARP is considered as follows. 1) In normal rat, CARP acted as a negative regulatory factor and its compensatory mechanism was activated to the action so that the expression regulation system of contractile protein genes was disarranged to cause enhanced expression of the contractile protein, resulting in cardiac dysfunction. On the other hand, the compensatory mechanism itself is broken down in heart failure; it is thus considered that the activity of CARP as a negative regulatory factor would lead directly to cardiac dysfunction. 2) It is reported that CARP has a possibility of regulating the function of contractile proteins on a protein level (Journal of Cell Biology, 153, 413-423, 2001). Assuming that CARP takes part not only in the expression of contractile proteins but also directly in maintenance of the sarcomeric structure, the results obtained herein that its overexpression destroyed the sarcomeric structure appear to be consistent.

Normally, CARP is a protein essential for the expression of contractile protein and the maintenance of sarcomeric structure. However, CARP appears to be one of heart failure causative genes, by which homeostasis of myocardial contraction is destroyed by its overexpression or excessive downregulation, leading to cardiac dysfunction.

### EXAMPLE 8

### Construction of the survey system using H9c2 cells

H9c2 cell is a cell line derived from rat cardiomyocytes and was purchased from ATCC. The cells were incubated in DMEM medium supplemented with 10% calf serum. After recovery of cells using trypsin, the cells were inoculated on a 12-well plate in 100000 cells/well, followed by incubation in a 10% calf serum-containing DMEM medium for 12 hours. Thereafter, the medium was exchanged with a fresh serum-free DMEM medium, to which norepinephrine was supplemented in 10 nM or 100 nM, and the CARP gene expression level was quantitatively determined by TaqMan PCR with passage of 4 hours and 8 hours. Also, the copy number of glycerol-3-phosphate dehydrogenase gene (G3PDH) was quantitatively determined in a similar manner, and the copy number of CARP gene calculated was corrected by G3PDH. The expression level of CARP gene was increased dependently on the dose of norepinephrine and showed approximately a 2-fold increase at maximum. Thus, it was revealed that the expression of CARP gene increased in response to hypertrophic stimuli even when using the cell line. According to this system, the CARP gene expression can be quantitatively determined in response to various hypertrophic stimuli or in the apoptosis induction stage, and thus the system can be used for screening a CARP gene expression regulator. Moreover, by performing reporter gene assay using a promoter of the CARP gene, a low variation rate can be changed to a high variation rate to achieve high sensitization, thus enabling to high throughput screening.

### INDUSTRIAL APPLICABILITY

The compound that regulates the activity of a protein having the same or substantially the same amino acid sequence as the amino acid sequence represented by SEQ ID NO:1, or its salt, or the neutralizing antibody regulating the activity of the above protein, can be used as preventive/therapeutic agents for diseases, e.g., heart diseases, etc. Also, the antisense DNA, etc. can suppress the expression of the protein having the same or substantially the same amino acid sequence as the amino acid sequence represented by SEQ ID NO:1 and, therefore, can be used as preventive/therapeutic agents for diseases, e.g., heart diseases, etc.

## Claims

1. A method of screening a compound that regulates the activity of a protein having the same or substantially the same amino acid sequence as the amino acid sequence represented by SEQ ID NO: 1, or its partial peptide, or a salt thereof, which comprises using a protein having the same or substantially the same amino acid sequence as the amino acid sequence represented by SEQ ID NO:1, or its partial peptide, or a salt thereof.

2. The screening method according to claim 1, wherein the protein having the same or substantially the same amino acid sequence as the amino acid sequence represented by SEQ ID NO:1, or its partial peptide, or a salt thereof, is expressed in the cytoplasm of a transformant transformed by a DNA containing a DNA encoding a protein having the same or substantially the same amino acid sequence as the amino acid sequence represented by SEQ ID NO:1, or its partial peptide, or a salt thereof.

3. A method of screening a CARP expression-promoting or inhibiting agent, which comprises assaying a level of CARP expressed, respectively, when a test compound is administered to a primary cardiomyocyte or myocardium-derived cell line (H9c2) capable of expressing CARP and when the test compound is not administered thereto.

4. A kit for screening a compound that regulates the activity of a protein having the same or substantially the same amino acid sequence as the amino acid sequence represented by SEQ ID NO:1, or its partial peptide, or a salt thereof, comprising a protein having the same or substantially the same amino acid sequence as the amino acid sequence represented by SEQ ID NO:1, or its partial peptide, or a salt thereof.

5. A compound or its salt that regulates the activity of a protein having the same or substantially the same amino acid sequence as the amino acid sequence represented by SEQ ID NO:1, or its partial peptide, or a salt thereof, which is obtainable by using the screening method according to claim 1 or the screening kit according to claim 4.

6. A compound or its salt that suppresses the activity of a protein having the same or substantially the same amino acid sequence as the amino acid sequence represented by SEQ ID NO:1, or its partial peptide, or a salt thereof, which is obtainable by using the screening method according to claim 1 or the screening kit according to claim 3.

7. A pharmaceutical comprising a compound or its salt that regulates the activity of a protein having the same or substantially the same amino acid sequence as the amino acid sequence represented by SEQ ID NO:1, or its partial peptide, or a salt thereof, which is obtainable by using the screening method according to claim 1 or the screening kit according to claim 4.

8. The pharmaceutical according to claim 7, which is a preventive/therapeutic agent for a heart disease.

9. A method of treating a heart disease which comprises administering the pharmaceutical according to claim 7 to a mammal.

10. Use of the compound or its salt according to claim 6 to prepare a preventive/therapeutic agent for a heart disease.

11. An antisense DNA having a base sequence complementary or substantially complementary to a DNA encoding a protein or its partial peptide having the same or substantially the same amino acid sequence as the amino acid sequence represented by SEQ ID NO:1.

12. A pharmaceutical comprising the antisense DNA according to claim 11.

13. A monoclonal antibody to a protein having the same or substantially the same amino acid sequence as the amino acid sequence represented by SEQ ID NO:1, or its partial peptide, or a salt thereof.

14. A diagnostic agent or pharmaceutical comprising an antibody to a protein having the same or substantially the same amino acid sequence as the amino acid sequence represented by SEQ ID NO:1, or its partial peptide, or a salt thereof.
